# EUROPEAN PATENT SPECIFICATION

(11) **EP 3 409 767 B1**
(45) Date of publication and mention of the grant of the patent: **21.10.2020**
(21) Application number: 17173411.4
(22) Date of filing: 30.05.2017
(51) Int. Cl.: C12N 9/24

(54) **MANNANASE VARIANTS**
MANNANASEVARIANTEN
VARIANTS DE LA MANNANASE

(43) Date of publication of application: 05.12.2018
(73) Proprietor: AB Enzymes Oy, 05200 Rajamäki (FI)
(72) Inventor: Leinonen, Taija, 80469 München (DE); Valtakari, Leena, 05200 Rajamäki (FI); Seefried, Michael, 64293 Darmstadt (DE); Juntunen, Kari, 05200 Rajamäki (FI); Puranen, Terhi, 05200 Rajamäki (FI)
(74) Representative: Espatent Oy

(56) References cited:
- WO-A1-2011/085747
- WO-A1-2012/079222
- WO-A1-2015/149641
- WO-A1-2017/021515
- "UNIPARC:UPI0003DC57EF", , 19 March 2014 (2014-03-19), XP002774974, Retrieved from the Internet: URL:http://www.uniprot.org/uniparc/UPI0003 DC57EF [retrieved on 2017-10-23]

## Description

### FIELD OF THE INVENTION

This invention relates to variants of mannanase enzyme. The variants are useful in industrial applications wherein degradation or modification of mannan is desired, such as in laundry and cleaning applications, in feed, food, pulp and paper and oil industry. The invention also provides useful mannanases enzymes, polynucleotides encoding these enzymes, enzyme compositions and methods for their production and use.

### BACKGROUND

Mannans are mannose containing polysaccharides found in various plants. Mannans are poorly soluble in aqueous environment and their physicochemical properties give rise to viscous dispersions. Additionally, mannans have high water binding capacity. All of these characteristics cause problems in several industries including brewing, baking, animal nutrition, and laundry and cleaning applications.

In plant-based diets different β-mannans are present and depending on their amounts and properties they can compromise nutrient digestion, microbial colonisation and growth performance. Enzymatic degradation of mannans reduces digesta viscosity of high water soluble mannans and leads to production of manno-oligosaccharides that may form water-insoluble linear mannans present in leguminoseae. Mannanase increases average daily gain, feed efficiency, weight uniformity and livability in all monogastric animals.

For animal feed applications, such as feed for monogastric animals with cereal diets, mannan is a contributing factor to viscosity of gut contents and it thereby adversely affects the feed digestibility and animal growth rate. For ruminants, mannan represents a substantial component of fiber intake and a more complete digestion of mannan would facilitate higher feed conversion efficiencies.

For laundry and cleaning applications enzyme compositions comprising mannanase can be used to degrade mannan. However, providing mannanases that are stable in varying storage and use conditions while still showing good mannan degrading activity is difficult.

N-linked glycosylation of proteins is a type of post-translational modification where a sugar molecule oligosaccharide known as glycan is attached to an amide nitrogen group of an asparagine (Asn, N) residue of a protein. This type of linkage is important for both the structure and function of enzymes and other proteins.

It is an object of the present invention to provide variants of mannanase having improved stability and exhibiting mannanase activity when applied in different industrial processes, as well as enzyme compositions for mannan degradation or modification.

### SUMMARY

According to the first aspect is provided a variant of mannanase having mannanase activity and a non-glycosylated amino acid at the position 283, wherein the variant is selected from the group consisting of
1) a polypeptide having at least 85% sequence identity to residues 27-331 of SEQ ID NO: 2;
2) a polypeptide encoded by a polynucleotide that hybridizes under high stringency conditions with
   a) nucleotides 79 - 993 of SEQ ID NO: 1 (*man7*)
   b) the full-length complement of a); and
3) a variant encoded by a polynucleotide having at least 95% sequence identity to the SEQ ID NO: 1 or the genomic DNA sequence thereof;
and wherein the amino acid numbering corresponds to the amino acid numbering of SEQ ID NO: 2 (Man7) full-length amino acid sequence containing a signal sequence, and wherein at least position N283, T285, or S285 is substituted by a residue which prevents N-linked glycosylation of the residue 283 when expressed in a host cell capable of N-linked glycosylation.

The present variant of mannanase is advantageous in having good stability and mannanase activity. The variant has different glycosylation pattern compared to a wild type mannanase, which may provide improved specific activity, stability and yield when produced in a host cell. Thus, the present variant of mannanase may provide improved yield in production and better performance in use. The stability of the variant is particularly good in detergents and at temperatures typically used in applications where mannan degradation is used, such as in laundry detergents.

According to the second aspect of the invention is provided an enzyme composition comprising the variant of mannanase of the first aspect and
a. at least one preservative selected for example from group consisting of organic acid, citric acid, ascorbic acid, benzoic acid and their salts and derivatives, sodium benzoate, benzoate, hydroxybenzoate and derivatives, sorbic acid, sodium sorbate, sorbate, salts, such as sodium chloride or potassium chloride, 1,2-Benzisothiazolin-3-one (BIT), or a combination thereof;
b. optionally at least one stabilizer selected from polyol, propylene glycol, polyethylene glycol, hexylene glycol, glycerol, a sugar, sugar alcohol, polysaccharide, lactic acid, boric acid, boric acid derivative, aromatic borate ester, 4-formylphenyl boronic acid, phenyl boronic acid derivative, peptide, surfactant, or a combination thereof;
c. optionally at least one enzyme selected from proteases, amylases, cellulases, lipases, xylanases, mannanases, cutinases, esterases, phytases, DNAses, pectinases, pectinolytic enzymes, pectate lyases, carbohydrases, arabinases, galactanases, xanthanases, xyloglucanases, laccases, peroxidases and oxidases with or without a mediator, or a combination thereof; and
d. optionally at least one filler selected from maltodextrin, flour, sodium chloride, sulfate, sodium sulfate, or a combination thereof.

As evidenced by the Examples, the variants comprised in the enzyme composition according to the invention have a structure and properties that allow production in recombinant host cells and make them useful in enzyme compositions for industrial applications. The enzyme composition is particularly good for detergent formulations because the variant of mannanase has good stability, wash performance and specific activity when used to degrade mannan in laundry and washing use.

According to the third aspect there is provided a detergent composition comprising the variant of mannanase of the first aspect or the enzyme composition of the second aspect.

The present detergent composition is advantageous in that it is efficient and economical in removing mannan containing stains.

According to another aspect is provided a use of, and a method of using, the present enzyme composition or the present variant of mannanase in a detergent.

According to the fourth aspect is provided a recombinant host cell comprising genetic elements that allow producing at least one recombinant polypeptide comprising the variant of mannanase of the first aspect.

According to the fifth aspect is provided a method for producing a recombinant polypeptide having mannanase activity and comprising:
a. cultivating a recombinant host cell of the fourth aspect, wherein
   the genetic elements comprise at least one control sequence which controls the production of the recombinant polypeptide in the recombinant host cell;
   the genetic elements optionally comprise at least one sequence encoding a signal sequence for transporting the recombinant polypeptide outside the host cell; and
   cultivating is carried out in conditions allowing production of the recombinant polypeptide; and
b. recovering the recombinant polypeptide.

The method provides an efficient way to produce a recombinant polypeptide comprising a variant of mannanase. Because the variant of mannanase is produced in a recombinant host cell, a production system is provided which can be optimized, tailored, and controlled in a desired manner. The variant of mannanase produced by the method may differ from natural mannanases at a structural and functional level. The variant of mannanase produced by the method has a glycosylation pattern, or other post translational modification, which causes differences in the structure and/or function when compared to a natural mannanase, such as a mannanase having similar amino acid sequence, or compared to a mannanase having the same amino acid sequence but produced in another host cell. In particular, when the variant is produced in a host cell capable of N-linked glycosylation, the variant has advantageously a non-glycosylated residue Asn283 resulting to improved properties. The variant of mannanase produced by the present method can be used as such, or formulated into a selected formulation.

According to another aspect is provided an enzyme preparation comprising a recombinant polypeptide having mannanase activity and obtainable by using the present host cell.

The enzyme preparation or the enzyme composition may further comprise other enzyme(s) selected from the group consisting of proteases, amylases, cellulases, lipases, xylanases, mannanases, cutinases, esterases, phytases, DNAses, pectinases, pectate lyases, pectinolytic enzymes, carbohydrases, arabinases, galactanases, xanthanases, xyloglucanases, laccases, peroxidases and oxidases with or without a mediator, as well as suitable additives selected from the group consisting of stabilizers, buffers, surfactants, bleaching agents, mediators, anti-corrosion agents, builders, anti-redeposition agents, optical brighteners, dyes, pigments, perfumes, caustics, abrasives and preservatives.

According to a sixth aspect is provided a method for degrading or modifying mannan containing material comprising treating said mannan containing material with an effective amount of the present enzyme composition or the present variant of mannanase.

According to the seventh aspect is provided an animal feed comprising the present enzyme composition or the present variant of mannanase, and at least one protein source of plant origin or a mannan containing product or by-product, and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

According to the eighth aspect is provided a feed supplement comprising the present enzyme composition or the present variant of mannanase; and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

The feed and the feed supplement improve nutritional value of feed compared to a feed without the variant. The present enzyme composition comprises the variant of mannanase, which has improved stability. The present enzyme composition and the present variant degrade mannan present in the feed and thereby make it more easily digestible for the animal. In particular for soybean meal containing feeds mannan-oligosaccharides that result from enzymatic digestion have a beneficial effects on the intestinal microbes, and consequently on the performance of the animals. The effect of the variants of mannanase can be enhanced by including xylanase to digest arabinoxylans present in corn soybean based diets. The present variants can also be used to modify rheological properties of wet feeds.

In an embodiment the feed may comprise animal protein, such as meat meal or bone meal.

According to another aspect is provided a use, and a method of using, the present animal feed or the present feed supplement in:
a. feeding animals;
b. improving weight gain of animals.

In an embodiment the animal is a monogastric animal or a ruminant. In another embodiment the animal is a broiler chicken, egg-laying chicken, swine, turkey, or an aquaculture organism such as fish. In another embodiment the animal is a ruminant.

According to a ninth aspect is provided a use of, and a method of using, the present variant or the present enzyme composition in oil drilling or hydro-fracturing.

The present enzyme composition and the present variant are advantageous in modifying rheological properties of oil drilling fluids and hydro-fracturing fluids, and to improve oil recovery.

According to an tenth aspect is provided a use of, and a method of using, the present variant or the present enzyme composition in processing coffee extract, fruit juice, pineapple juice, or soya milk.

Using the present variant and the present enzyme composition is advantageous in processing coffee extract because it reduces viscosity of the coffee extract.

Using the present variant and the present enzyme composition is advantageous in processing and manufacturing fruit juice because it lowers viscosity and improves filtration rate, stability and helps to extract fruit components.

Using present variant and the present enzyme composition is advantageous in processing and manufacturing soya milk because it improves yield, colour, protein content and taste of soya milk.

Herein is also disclosed a nucleic acid molecule encoding the present variant of mannanase.

Herein is also disclosed a vector comprising the present nucleic acid molecule.

Herein is also disclosed a functional fragment of the present variant of mannanase, and a polynucleotide encoding it.

Herein is also disclosed that the disclosed sequence information relating to a polynucleotide sequence encoding a mannanase of the invention can be used as a tool to identify other homologous mannanases. For instance, polymerase chain reaction (PCR) can be used to amplify sequences encoding other homologous mannanases from a variety of biological sources. In addition, genome mining approaches can be used to identify sequences encoding other homologous mannanases from genome databases.

### BRIEF DESCRIPTION OF THE FIGURES

**Fig 1** shows schematic representation of expression cassette.
**Fig 2** shows the relative specific activity of the variants (TBH5, TBH6, TBH9, TBH10 and TBH11) compared to the wild type Man7 mannanase (produced in *Trichoderma*).
**Fig 3A-B** describe the stain removal performance of variants and wild type Man7 (produced in *Trichoderma*) as an increase of lightness (sum of ΔL*of 3 stains) in the presence of 4,4 g/l of Commercial heavy duty liquid detergent A at 40°C, 16 °dH, 60 min, pH approx. 8.3 and enzymes dosed as activity units (MNU) per wash liquor.

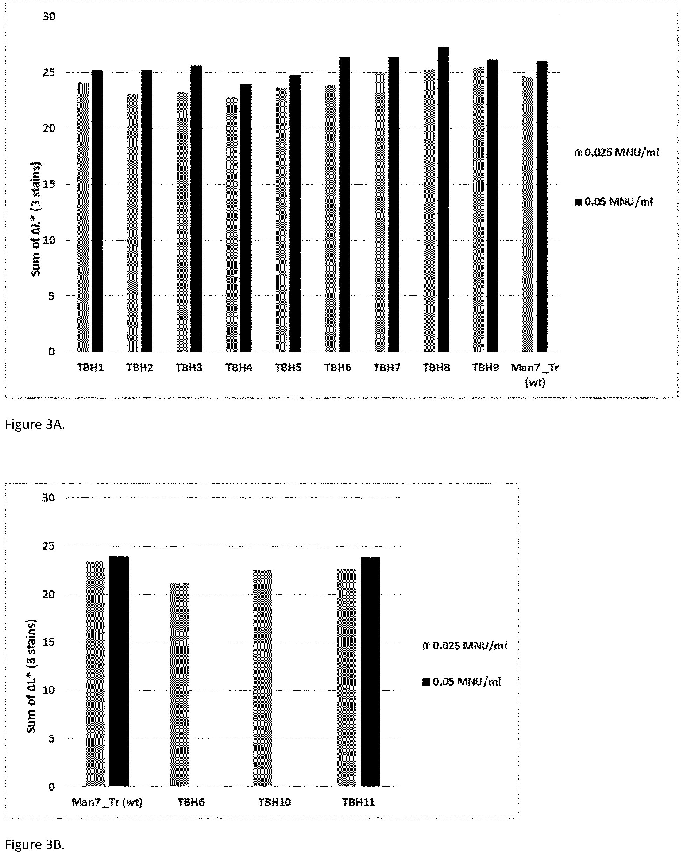

**Fig 3A** shows variants TBH1, TBH2, TBH3, TBH4, TBH5, TBH6, TBH7, TBH8, TBH9 and wild type.
**Fig 3B** shows variants TBH6, TBH10, TBH11 and wild type.

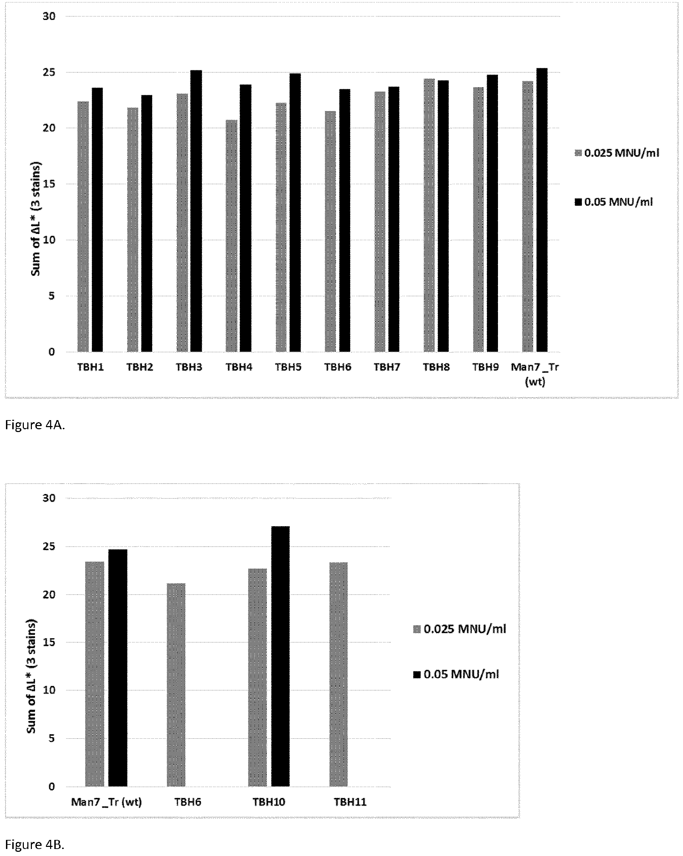

**Fig 4A-B** describe the stain removal performance of variants and wild type Man7 (produced in *Trichoderma*) as an increase of lightness (sum of ΔL*of 3 stains) in the presence of 3,8 g/l of Commercial color detergent powder at 40°C, 16 °dH, 60 min, pH approx. 10 and enzymes dosed as activity units (MNU) per wash liquor.
**Fig 4A** shows variants TBH1, TBH2, TBH3, TBH4, TBH5, TBH6, TBH7, TBH8, TBH9 and wild type Man7
**Fig 4B** shows variants TBH6, TBH10, TBH11 and wild type Man7.

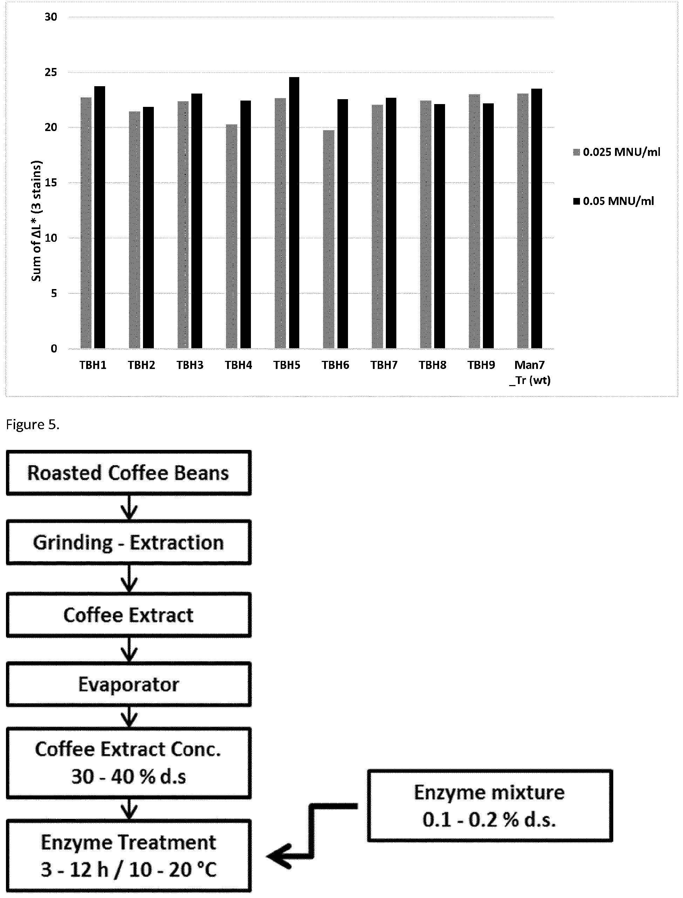

**Fig 5** describes the stain removal performance of variants TBH1, TBH2, TBH3, TBH4, TBH5, TBH6, TBH7, TBH8, TBH9 and wild type Man7 (produced in *Trichoderma*) as an increase of lightness (sum of ΔL*of 3 stains) in the presence of 4,2 g/l of Commercial bleach detergent powder at 40°C, 16 °dH, 60 min, pH approx. 9.5 and enzymes dosed as activity units (MNU) per wash liquor.
**Fig 6** shows a flow chart of instant coffee production involving use of the mannanase variants of the invention.

### DEPOSITS

The following strain depositions according to the Budapest Treaty on the International Recognition of Deposit of Microorganisms for the Purposes of Patent Procedure were made:
The *E.coli* strain RF12379 including the plasmid pALK4434 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32425.

The *E.coli* strain RF12380 including the plasmid pALK4435 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32426.

The *E.coli* strain RF12381 including the plasmid pALK4436 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32427.

The *E.coli* strain RF12382 including the plasmid pALK4437 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32428.

The *E.coli* strain RF12383 including the plasmid pALK4438 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32429.

The *E.coli* strain RF12384 including the plasmid pALK4439 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32430.

The *E.coli* strain RF12385 including the plasmid pALK4440 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32431.

The *E.coli* strain RF12386 including the plasmid pALK4441 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32432.

The *E.coli* strain RF12387 including the plasmid pALK4442 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32433.

The *E.coli* strain RF12456 including the plasmid pALK4432 was deposited at the Deutsche Sammlung von Mikroorganismen und Zellkulturen GmbH (DSMZ), Inhoffenstrasse 7 b, D-38124 Braunschweig, Germany on 18 May, 2017 and assigned accession number DSM 32518.

The *E.coli* strain RF12457 including the plasmid pALK4433 was deposited at the Deutsche Sammlung von Mikroorganismen und Zellkulturen GmbH (DSMZ), Inhoffenstrasse 7 b, D-38124 Braunschweig, Germany on 18 May, 2017 and assigned accession number DSM 32519.

### SEQUENCE LISTINGS

SEQ ID NO: 1 DNA sequence of the *man7*
SEQ ID NO: 2 Full-length amino acid sequence of the Man7
SEQ ID NO: 3 Deduced amino acid sequence (mature) of the Man7
SEQ ID NO: 4 Core amino acid sequence of the Man7, no CBM
SEQ ID NO: 5 Synthetic gene sequence of the variant *tbh1*
SEQ ID NO: 6 Deduced amino acid sequence (mature) of the variant TBH1
SEQ ID NO: 7 Synthetic gene sequence of the variant *tbh2*
SEQ ID NO: 8 Deduced amino acid sequence (mature) of the variant TBH2
SEQ ID NO: 9 Synthetic gene sequence of the variant *tbh3*
SEQ ID NO: 10 Deduced amino acid sequence (mature) of the variant TBH3
SEQ ID NO: 11 Synthetic gene sequence of the variant *tbh4*
SEQ ID NO: 12 Deduced amino acid sequence (mature) of the variant TBH4
SEQ ID NO: 13 Synthetic gene sequence of the variant *tbh5*
SEQ ID NO: 14 Deduced amino acid sequence (mature) of the variant TBH5
SEQ ID NO: 15 Synthetic gene sequence of the variant *tbh6*
SEQ ID NO: 16 Deduced amino acid sequence (mature) of the variant TBH6
SEQ ID NO: 17 Synthetic gene sequence of the variant *tbh7*
SEQ ID NO: 18 Deduced amino acid sequence (mature) of the variant TBH7
SEQ ID NO: 19 Synthetic gene sequence of the variant *tbh8*
SEQ ID NO: 20 Deduced amino acid sequence (mature) of the variant TBH8
SEQ ID NO: 21 Synthetic gene sequence of the variant *tbh9*
SEQ ID NO: 22 Deduced amino acid sequence (mature) of the variant TBH9
SEQ ID NO: 23 Synthetic gene sequence of the variant *tbh10*
SEQ ID NO: 24 Deduced amino acid sequence (mature) of the variant TBH10
SEQ ID NO: 25 Synthetic gene sequence of the variant *tbh11*
SEQ ID NO: 26 Deduced amino acid sequence (mature) of the variant TBH11

### DETAILED DESCRIPTION

Mannan refers to polysaccharides consisting of a mannose backbone linked together by β-1,4-linkages with side-chains of galactose attached to the backbone by α-1,6-linkages. Mannans comprise plant-based material such as guar gum and locust bean gum. Glucomannans are polysaccharides having a backbone of more or less regularly alternating β-1,4 linked mannose and glucose, galactomannans and galactoglucomannans are mannans and glucomannans with alpha-1,6 linked galactose side branches.

The term "functional fragment" or "effective fragment" means a fragment or portion of SEQ ID NO: 2 (Man7) variant that retains about the same enzymatic function or effect.

The term "mannanase variants" and "variant of mannanase" means any mannanase molecule obtained by site-directed or random mutagenesis, insertion, substitution, deletion, recombination and/or any other protein engineering method, which leads to mannanases that differ in their amino acid sequence from the parent mannanase, i.e. a wild-type mannanase. The terms "wild type mannanase", "wild type enzyme", "wild type", or "wt" in accordance with the disclosure describe a mannanase enzyme with an amino acid sequence found in nature or a fragment thereof.

The term "catalytic activity" or "activity" describes quantitatively the conversion of a given substrate under defined reaction conditions. The term "residual activity" is defined as the ratio of the catalytic activity of the enzyme under a certain set of conditions to the catalytic activity under a different set of conditions. Therefore the residual activity aᵢ is given by aᵢ=vᵢ/v₀ where v denotes any measure of catalytic activity and aᵢ *100 is the relative activity in percent. The term "specific activity" describes quantitatively the catalytic activity per amount of enzyme under defined reaction conditions.

The term "proteolytic stability" describes the property of a protein to withstand a limited exposure to proteases under conditions where the proteases are active, without losing activity under conditions where its activity can be measured.

As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78.

As used herein, "isolated" means a substance in a form or environment that does not occur in nature. Non-limiting examples of isolated substances include (1) any non-naturally occurring substance, (2) any substance including any enzyme, variant, nucleic acid, protein, peptide or cofactor, that is at least partially removed from one or more or all of the naturally occurring constituents with which it is associated in nature; (3) any substance modified by the hand of man relative to that substance found in nature, such as a variant; or (4) any substance modified by increasing or decreasing the amount of the substance relative to other components with which it is naturally associated (e.g., recombinant production in a host cell; one or multiple copies of a gene encoding the substance; and use of an alternative promoter to the promoter naturally associated with the gene encoding the substance). In an embodiment a polypeptide, enzyme, variant, polynucleotide, host cell or composition of the invention is isolated.

As used herein, the term "comprising" includes the broader meanings of "including", "containing", and "comprehending", as well as the narrower expressions "consisting of" and "consisting only of".

As used herein, "variant" means a sequence or a fragment of a sequence (nucleotide or amino acid) inserted, substituted or deleted by one or more nucleotides/amino acids, or which is chemically modified. In an embodiment the term variant also includes a recombinant mannanase enzyme.

As used herein, "conservative amino acid substitution" is one in which the amino acid residue is replaced with an amino acid residue having a similar side chain. Families of amino acid residues having similar side chains have been defined in the art. In an embodiment the conservative amino acids in the present description refer to the amino acids within following groupings: Hydrophobic (F W Y H K M I L V A G C); Aromatic (F W Y H); Aliphatic (I L V); Polar (W Y H K R E D C S T N Q); Charged (H K R E D); Positively charged (H K R); Negatively charged (E D); Small (V C A G S P T N D); Tiny (A G S). Thus, a conservative substitution occurs when an amino acid is substituted with an amino acid in the same group.

In an embodiment the substitution is a substitution with at least one amino acid residue. In a further embodiment the at least amino acid is Ala.

As used herein, a "non-conservative amino acid substitution" is one in which an amino acid is substituted with an amino acid in a different group as defined above. The non-conservative substitution may result into a change of an amino acid to another amino acid with different biochemical properties, such as charge, hydrophobicity and/or size. In an embodiment the non-conservative substitution changes at least one property of the variant, such as stability, glycosylation pattern, folding, structure, activity, or affinity.

N-linked glycosylation process occurs in eukaryotes, but very rarely in bacteria. The attachment of a glycan residue to a protein requires the recognition of a consensus sequence. N-linked glycans are almost always attached to an asparagine (Asn) side chain that is present as a part of Asn-X-Ser/Thr consensus sequence, wherein X is any amino acid except for proline (Pro). The inventors have found that a non-glycosylated Asn side chain structurally close to the active site of the mannanase is important for obtaining a variant with good mannan degrading performance. Without being bound to any theory, glycan sugars are polar molecules and when attached to an Asn, they locate on the surface of the protein causing structural changes in the glycosylated Asn and in its vicinity. Site directed mutagenesis of Asn or Ser/Thr residues in the Asn-Xaa-Thr(Ser) consensus sequence can be used to prevent glycosylation of the desired N-linked glycosylation sites in the variant of the first aspect of the invention.

In an embodiment the variant has a substitution of an amino acid by a residue which prevents N-linked glycosylation of the residue 283 when expressed in a host cell capable of N-linked glycosylation.

In an embodiment the present variant comprises at least one Asn-X-Ser/Thr consensus sequence.

In an embodiment the present variant comprises Pro residue in the location X of the at least one Asn-X-Ser/Thr consensus sequence.

In an embodiment the substitution is either a conservative or a non-conservative substitution.

As used herein, a "peptide" and a "polypeptide" are amino acid sequences including a plurality of consecutive polymerized amino acid residues. For purpose of this invention, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

The term "polynucleotide" denotes a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules.

As used herein, "modification", "modified", and similar terms in the context of polynucleotides refer to modification in a coding or a non-coding region of the polynucleotide, such as a regulatory sequence, 5' untranslated region, 3' untranslated region, up-regulating genetic element, down-regulating genetic element, enhancer, suppressor, promoter, exon, or intron region. The modification may in some embodiments be only structural, having no effect on the biological effect, action or function of the polynucleotide. In other embodiments the modification is a structural modification, which provides a change in the biological effect, action or function of the polynucleotide. Such a modification may enhance, suppress or change the biological function of the polynucleotide.

As used herein, "identity" means the percentage of exact matches of amino acid residues between two aligned sequences over the number of positions where there are residues present in both sequences. When one sequence has a residue with no corresponding residue in the other sequence, the alignment program allows a gap in the alignment, and that position is not counted in the denominator of the identity calculation. Identity is a value determined with the Pairwise Sequence Alignment tool EMBOSS Needle at the EMBL-EBI website (www.ebi.ac.uk/Tools/psa/emboss needle/).

As used herein, low stringency conditions mean for probes of at least 100 nucleotides in length conditions corresponding to hybridizing at prehybridisation and hybridisation at 55 °C in 5× SSC, 0.1 % N-lauroylsarcosine, 0.02 % SDS, 1% blocking reagent (Roche 11 096 176 001), following standard Southern blotting procedures for 12 to 24 hours. The carrier material is finally washed two to three times each for 15 minutes using 2X SSC, 0.1% SDS at 55°C.

As used herein, high stringency conditions mean for probes of at least 100 nucleotides in length conditions corresponding to hybridizing at prehybridisation and hybridization at 65°C in 5× SSC, 0.1% N-lauroylsarcosine, 0.02% SDS, 1% blocking reagent (Roche 11 096 176 001), following standard Southern blotting procedures for 12 to 24 hours. The carrier material is finally washed two to three times each for 15 minutes using 0.1X SSC, 0.1% SDS at 65°C.

As used herein, "host cell" means any cell type that is susceptible to transformation, transfection, transduction, mating, crossing or the like with a nucleic acid construct or expression vector comprising a polynucleotide. The term "host cell" encompasses any progeny that is not identical due to mutations that occur during replication. Non-limiting examples of a host cell are fungal cells, filamentous fungal cells from Division *Ascomycota,* Subdivision *Pezizomycotina*; preferably from the group consisting of members of the Class *Sordariomycetes,* Subclass *Hypocreomycetidae,* Orders *Hypocreales* and *Microascales* and *Aspergillus, Chrysosporium, Myceliophthora and Humicola*; more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium*; more preferably from the group consisting of *Trichoderma reesei (Hypocrea jecorina), T. citrinoviridae, T. longibrachiatum, T. virens, T. harzianum, T. asperellum, T. atroviridae, T. parareesei,* , *Fusarium oxysporum, F. gramineanum, F. pseudograminearum, F. venenatum, Gibberella fujikuroi, G. moniliformis,* G. zeaea, *Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,* most preferably *Trichoderma reesei.* Non-limiting examples of a host cell are bacterial cells, preferably gram positive Bacilli (e.g. *Bacillus subtilis, B. licheniformis, B. megaterium, B. amyloliquefaciens, B. pumilus*), gram-negative bacteria (e.g. *Escherichia coli*), actinomycetales (e.g. *Streptomyces* sp.) and yeasts (e.g. *Saccharomyces cerevisiae, Pichia pastoris, Yarrowia lipolytica).*

In an embodiment the host cell is a fungal cell, preferably a filamentous fungal cell, such as *Trichoderma* or *Trichoderma reesei.* In an embodiment the host cell is a bacterial cell, preferably a gram positive *Bacillus* cell, such as *B. subtilis, B. licheniformis, B. megaterium, B. amyloliquefaciens, B. pumilus.*

In an embodiment the host cell is capable of N-linked glycosylation.

A "recombinant cell" or "recombinant host cell" refers to a cell or host cell, which has been genetically modified or altered to comprise a nucleic acid sequence which is not native to said cell or host cell. The genetic modification may comprise integrating the polynucleotide in the genome of the host cell. The polynucleotide may also be exogenous in the host cell. In an embodiment the present host cell is a recombinant host cell.

As used herein, "expression" includes any step involved in the production of a polypeptide in a host cell including, but not limited to, transcription, translation, post-translational modification, and secretion. Expression may be followed by harvesting, i.e. recovering, the host cells or the expressed product.

The term "expression vector" denotes a DNA molecule, linear or circular, that comprises a segment encoding a polypeptide of interest operably linked to additional segments that provide for its transcription. Such additional segments may include promoter and terminator sequences, and may optionally include one or more origins of replication, one or more selectable markers, an enhancer, a polyadenylation signal, carrier and the like. Expression vectors are generally derived from plasmid or viral DNA, or may contain elements of both. The expression vector may be any expression vector that is conveniently subjected to recombinant DNA procedures, and the choice of vector will often depend on the host cell into which the vector is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated. In an embodiment the present vector is an expression vector.

The term "recombinant produced" or "recombinantly produced" used herein in connection with production of a polypeptide or protein is defined according to the standard definition in the art.

The term "obtained from" and "obtainable" as used herein in connection with a specific microbial source means that the polynucleotide is expressed by the specific source (homologous expression), or by a cell in which a gene from the source has been inserted (heterologous expression).

The term "enzyme composition" means either a conventional enzymatic fermentation product, possibly isolated and purified, from a single species of a microorganism, such preparation usually comprising a number of different enzymatic activities; or a mixture of monocomponent enzymes, preferably enzymes derived from bacterial or fungal species by using conventional recombinant techniques, which enzymes have been fermented and possibly isolated and purified separately and which may originate from different species, preferably fungal or bacterial species or the fermentation product of a microorganism which acts as a host cell for production of a recombinant mannanase, but which microorganism simultaneously produces other enzymes.

The term "operably linked", when referring to DNA segments, denotes that the segments are arranged so that they function in concert for their intended purposes, e.g. transcription initiates in the promoter and proceeds through the coding segment to the terminator.

The term "promoter" denotes a portion of a gene containing DNA sequences that provide for the binding of RNA polymerase and initiation of transcription. Promoter sequences are commonly, but not always, found in the 5' non-coding regions of genes.

The term "secretory signal sequence" or "signal sequence" denotes a DNA sequence that encodes a polypeptide (a "secretory peptide") that, as a component of a larger polypeptide, directs the larger polypeptide through a secretory pathway of a host cell in which it is produced. The secretory signal sequence can be native or it can be replaced with secretory signal sequence or carrier sequence from another source. Depending on the host cell, the larger peptide may be cleaved to remove the secretory peptide during transit through the secretory pathway.

The term "core region" or "catalytic domain" denotes a domain of an enzyme, which may or may not have been modified or altered, but which has retained at least part of its original activity. The core region of a mannanase according to the invention corresponds to the amino acids aligned with the amino acids 27-331 of Man7, SEQ ID NO: 2.

By the term "linker" or "spacer" is meant a polypeptide comprising at least two amino acids which may be present between the domains of a multidomain protein, for example an enzyme comprising an enzyme core and a binding domain such as a carbohydrate binding module (CBM) or any other enzyme hybrid, or between two proteins or polypeptides produced as a fusion polypeptide, for example a fusion protein comprising two core enzymes. For example, the fusion protein of an enzyme core with a CBM is provided by fusing a DNA sequence encoding the enzyme core, a DNA sequence encoding the linker and a DNA sequence encoding the CBM sequentially into one open reading frame and expressing this construct.

Efficient amount means an amount, which is sufficient to degrade mannose in the selected application.

The following abbreviations are used for amino acids:

| | | |
|---|---|---|
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |

Substitutions are described using of the following nomenclature: amino acid residue in the protein scaffold; position; substituted amino acid residue(s). According to this nomenclature the substitution of, for instance, a serine residue for a glycine residue at position 20 is indicated as Ser20Gly or S20G.

The terms "detergent composition" and "detergent" include, unless otherwise indicated, solid, granular or powder-form all-purpose or heavy-duty washing agents, especially cleaning detergents; liquid, gel or paste-form all-purpose washing agents, especially the so- called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners; metal cleaners; as well as cleaning auxiliaries such as bleach additives and "stain-stick" or pre-treat types. The terms "detergent", "detergent composition" and "detergent formulation" are used in reference to mixtures, which are intended for use in a wash medium for the cleaning of soiled objects. In some embodiments, the term is used in reference to laundering fabrics and/or garments (e.g., "laundry detergents"). In alternative embodiments, the term refers to other detergents, such as those used to clean dishes, cutlery, etc. (e.g., "dishwashing detergents"). It is not intended that the present invention be limited to any particular detergent formulation or composition. It is intended that in addition to the mannanases according to the invention, the term encompasses detergents that may contain e.g., surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, hydrotropes, fabric hueing agents, dispersants, dye transfer inhibiting agents, fluorescent whitening agents, soil release polymers, anti-redepositions agents, anti-shrink agents, anti-wrinkling agents, bactericides, binders, carriers, dyes, enzyme stabilizers, fabric softeners, fillers, foam regulators, perfumes, pigments, sod suppressors, solvents, and structurants for liquid detergents, structure elasticizing agents, enzyme inhibitors or stabilizers, enzyme activators, transferase(s), hydrolytic enzymes, oxido reductases, bluing agents and fluorescent dyes, antioxidants, and solubilizers.

The term "textile" means any textile material including yarns, yarn intermediates, fibers, non-woven materials, natural materials, synthetic materials, and any other textile material, fabrics made of these materials and products made from fabrics (e.g., garments, linen and other articles). The textile or fabric may be in the form of knits, wovens, denims, non-wovens, felts, yarns, and towelling. The textile may be cellulose based, such as natural cellulosics including cotton, flax/linen, jute, ramie, sisal or coir or manmade cellulosics (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, for example stained household laundry. When the term fabric or garment is used it is intended to include the broader term textiles as well.

The term "stability" includes storage stability and stability during use, e.g. during a wash process (in wash stability) and reflects the stability of the mannanase according to the invention as a function of time, e.g. how much activity is retained when the mannanase is kept in solution, in particular in a detergent solution. The stability is influenced by many factors, e.g. pH, temperature, detergent composition e.g. proteases, stabilizers, builders, surfactants etc. The mannanase stability may be measured using the 'activity assay' as described in examples.

"Mannanase activity" as used herein refers to the mannan degrading activity of a polypeptide. Degrading or modifying as used herein means that mannose units are hydrolyzed from the mannan polysaccharide by the mannanase. The mannan degrading activity of the polypeptides according to present invention can be tested according to standard test procedures known in the art. Example 4 provides an example of a standard method for determining mannanase activity.

In an embodiment the present variant has at least position N283, T285, or S285; preferably T285 or S285; most preferably T285; substituted by a residue, which prevents N-linked glycosylation of the residue 283 when expressed in a host cell capable of N-linked glycosylation.

In an embodiment the present variant has at least one further substitution at a position corresponding to the position below:
229, 283, 285, 300, 340, 400, 419, 433 or 446; or
S229, N283, T285, S285, N300, N340, S400, N419, S433 or N446.

In an embodiment the substitution comprises a substitution at said position to an amino acid selected from Ala, Arg, Asn, Asp, Cys, Gln, Glu, Gly, His, Ile, Leu, Lys, Met, Phe, Pro, Ser, Thr, Trp, Tyr, and Val. Preferably the substitution is a substitution to an amino acid other than Ser or Thr.

In an embodiment the variant comprises a P residue in the position 284 and/or 286. A substitution to a P residue in any or both of the above positions may cause a structural change in the variant, which prevents N-linked glycosylation.

In an embodiment in the present variant the position T285 or S285 is substituted to a residue other than T or S.

In an embodiment in the present variant the position T285 or S285 is substituted to an alanine.

In an embodiment the substitution is a conservative or non-conservative substitution resulting into an altered glycosylation of the variant when produced in a host cell capable of glycosylation, preferably N-linked glycosylation.

In an embodiment is provided a variant of mannanase comprising a polypeptide having at least 85% sequence identity to residues 27-331 of SEQ ID NO: 2, and a non-glycosylated Asn residue at position 283 when produced in a host cell capable of N-linked glycosylation.

In an embodiment the present variant comprises at least one additional glycosylated N site, wherein the position corresponding to the N283 is not glycosylated. Such a variant is obtainable by producing the variant in a host cell which is capable of N-glycosylation, resulting into at least partial glycosylation of the other N-glycosylation sites, but wherein the N-glycosylation of N283 is inhibited.

In a further embodiment the present variant does not comprise any glycosylated N site. Such a variant is obtainable by producing the variant in a host cell which is not capable of N-glycosylation, or by producing the variant in a host cell capable of glycosylation but wherein the variant has been engineered such that the other N-glycosylation sites are not glycosylated, resulting into inhibited glycosylation of the variant.

In an embodiment the variant has a predicted molecular weight between 50000 and 51000, preferably between 50750 and 50970, without including the signal sequence.

In an embodiment the variant has a predicted pl between 4.5 and 4.8, preferably between 4.6 and 4.75.

Prediction of pl or molecular weight of the variant can be carried out as described in the Table 3.

In a further embodiment of the invention the variant has mannanase activity. In an embodiment the variant has increased specific activity compared to a wild type mannanase when produced in a eukaryotic host cell. The mannanases comprised in the present enzyme composition of the invention are suitable for degrading and modifying mannan containing material in various chemical environments, preferably in detergent compositions.

In an embodiment of the first aspect the present enzyme composition is in the form of a liquid composition or a solid composition such as solution, dispersion, paste, powder, granule, granulate, coated granulate, tablet, cake, crystal, crystal slurry, gel or pellet.

The present invention furthermore relates to different uses of the present enzyme composition, such as for degrading mannan and for use in a laundry process.

The present enzyme composition can also be used in cleaning agents or boosters that are added on top of the detergent during or before the wash and that are for example in the form of liquid, gel, powder, granules or tablets. Enzyme composition and detergent components may also be soaked in a carrier like textiles.

In one embodiment of the present invention the enzyme composition further comprises one or more additional enzymes selected from the group consisting of protease, lipase, cutinase, amylase, carbohydrase, cellulase, pectinase, pectatelyase, pectinolytic enzyme, esterase, phytase, mannanase, arabinase, galactanase, xylanase, oxidase, xanthanase, xyloglucanase, DNAse, laccase, and/or peroxidase, preferably selected from the group consisting of proteases, amylases, cellulases and lipases.

The present enzyme composition comprising mannanase and an additional enzyme is advantageous in providing synergistic effect. Such additional enzymes are desired when the present enzyme composition comprising mannanase is used in detergents e.g. when washing stains. Particularly advantageous synergistic enzymes that work with mannanase in detergents are amylases, proteases and cellulases, or a combination thereof, such as a composition comprising mannanase, amylase and protease.

In an embodiment the present detergent composition is in a form of a bar, a homogenous tablet, a tablet having two or more layers, a pouch having one or more compartments, a regular or compact powder, bar, tablet, a granule, granulate, liquid, granulate, a paste, a gel, or a regular, compact or concentrated liquid. In one embodiment the detergent composition can be a laundry detergent composition, preferably a liquid or solid laundry detergent composition.

In one embodiment of the present invention the present detergent composition further comprises one or more additional enzyme selected from the group consisting of protease, lipase, cutinase, amylase, carbohydrase, cellulase, pectinase, pectatelyase, pectinolytic enzyme, esterase, mannanase, arabinase, galactanase, xylanase, oxidase, xanthanase, xyloglucanase, laccase, DNAse and/or peroxidase, preferably selected from the group consisting of proteases, amylases, cellulases and lipases.

The present invention furthermore relates to the use of, and a method of using, the enzyme composition or the detergent composition as herein disclosed for degrading mannan.

In a further embodiment the present invention relates to the use of, and a method of using, the enzyme composition or the detergent composition as herein disclosed in a laundry process.

The present invention furthermore relates to a method for removing a stain from a surface, comprising contacting the surface with the enzyme composition or the detergent composition as herein disclosed.

The present invention also relates to a method for degrading mannan comprising applying the enzyme composition or the detergent composition as herein disclosed to mannan, preferably wherein the mannan is on a surface of a textile, or at least partially embedded in a textile.

In general the properties of the selected enzyme(s) should be compatible with the selected detergent, (i.e., pH-optimum, compatibility with other enzymatic and non-enzymatic ingredients, etc.), and the enzyme(s) should be present in effective amounts.

A composition for use in solid laundry detergent, for example, may include 0.000001% - 5%, such as 0.000005-2%, such as 0.00001%-1%, such as 0.00001%-0.1% of enzyme protein by weight of the composition.

A composition for use in laundry liquid, for example, may include 0.000001%-3%, such as 0.000005%-1%, such as 0.00001%-0.1% of enzyme protein by weight of the composition.

A composition for use in automatic dishwash, for example, may include 0.000001%-5%, such as 0.000005%-2%, such as 0.00001%-1%, such as 0.00001%-0.1% of enzyme protein by weight of the composition.

The additional components a-d of the second aspect of the invention provide improved properties for the present enzyme composition. The enzyme composition is compatible with the additional components and improves applicability of the enzyme composition in various uses.

Salts, such as sodium chloride and sodium sulfate function as drying aids.

In an embodiment the variant of mannanase comprises a core region.

In an embodiment the variant of mannanase comprises a CBM.

Providing mannanases that retain activity in temperatures above ambient temperature is advantageous for applications wherein mannan degradation is required in such conditions. Further, the mannanases according to invention may have good stability and activity in alkaline conditions, which is advantageous in detergent use and in biomass processing.

In an embodiment the variant of mannanase has an amino acid sequence with at least, or about, 85%, 86%, 87%, 88%, 89%, 90%, 91%, 92%, 93%, 94%, 95%, 96%, 97%, 98%, 99% or 100% sequence identity to SEQ ID NO:2.

In an embodiment the variant of mannanase has an amino acid sequence with at least 90% sequence identity to SEQ ID NO:2.

In an embodiment the mannanase enzyme has an amino acid sequence which is not 100% identical to SEQ ID NO: 2.

In an embodiment of the third aspect the host cell is selected from the group consisting of:
fungal cells,
filamentous fungal cells from Division *Ascomycota,* Subdivision *Pezizomycotina*; preferably from the group consisting of members of the Class *Sordariomycetes,* Subclass *Hypocreomycetidae,* Orders *Hypocreales* and *Microascales* and *Aspergillus, Chrysosporium, Myceliophthora* and *Humicola*;
more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium*;
more preferably from the group consisting of *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis,* G. zeaea, *Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,*
bacterial cells, preferably gram positive Bacilli such as *B. subtilis, B. licheniformis, B. megaterium, B. amyloliquefaciens, B. pumilus,* gram negative bacteria such as *Escherichia coli,* actinomycetales such as *Streptomyces* sp., and
yeasts, such as *Saccharomyces cerevisiae, Pichia pastoris, Yarrowia lipolytica,*
most preferably *Trichoderma reesei.*

In an embodiment the host cell is an eukaryotic host cell capable of N-linked glycosylation. In a preferred embodiment the host cell is *Trichoderma reesei.*

The recombinant host cell can be used to produce the variant of mannanase and to carry a polynucleotide encoding it. The recombinant host cell is useful also in preparation of variants of mannanase with different properties. For example, a host cell can be selected, which provides post-translational modifications beneficial for stability or activity, or which facilitates post-processing and formulation of the variant of mannanase produced in the host cell.

In an embodiment the present enzyme composition comprises the recombinant host cell of the second aspect.

In an embodiment the present variant of mannanase is a recombinant polypeptide, which is a fusion protein.

In an embodiment the present recombinant polypeptide is a fusion protein, which further comprises at least one of:
an amino acid sequence providing a secretory signal sequence;
an amino acid sequence which facilitates purification, such as an affinity tag, His-tag;
an amino acid sequence which enhances production, such as an amino acid sequence which is a carrier, such as CBM;
an amino acid sequence having an enzyme activity; and
an amino acid sequence providing for the fusion protein with binding affinity, such as a carbohydrate binding moiety.

The CBM, carbohydrate binding moiety, as a carrier is advantageous e.g. in *Trichoderma* production.

In an embodiment the host cell is non-pathogenic. This is particularly advantageous for using the host cell in feed, and in detergent applications such as in home laundry detergents.

In an embodiment of the fifth aspect the mannan containing material is selected from plant based material, textile, waste water, sewage, oil or a combination thereof.

In an embodiment the mannan containing material is textile material or fabric.

In another embodiment the mannan containing material is recycled waste paper; mechanical pulp, chemical pulp, semi chemical pulp, Kraft or other paper-making pulps; fibres subjected to a retting process; or guar gum or locust bean gum containing material.

In another embodiment degradation or modifying is carried out in an aqueous environment wherein mannanase shows activity.

In a preferred embodiment the mannan containing material, which is degraded or modified in the method, is on a textile or a fabric optionally with mannan stains. By degrading mannan attached to the textile or fabric, dirt or soil bound to mannan is released and not capable of binding again to the mannan or mannan stains. The textile or fabric can be of any material, for example cotton, flax/linen, jute, ramie, sisal or coir or manmade cellulosics (e.g. originating from wood pulp) including viscose/rayon, modal, cellulose acetate fibers (tricell), lyocell, cupro or blends thereof.

In an embodiment the present feed comprises or consists of maize and soybean meal.

In an embodiment the protein source of plant origin comprises or consist of soy, cereal such as barley, wheat, rye, oats, or maize.

In an embodiment the mannan containing product or by-product comprises or consists of palm kernel, guar meal or copra meal.

In an embodiment the present animal feed or the present feed supplement is formulated in the form of a wet composition or a dry composition.

In an embodiment the composition comprising at least one variant of mannanase enzyme is used in pulp and paper industry, biobleaching, fiber modification, drainage improvement and in the oil industry, i.e. in oil drilling or oil-servicing industry for hydro-fracturing or controlling the viscosity of drilling fluids.

In an embodiment the composition comprising at least one variant of mannanase is used in textile and detergent industry, biomass processing and biomass hydrolysis, preferably in biofuel, starch, pulp and paper, food, baking, feed or beverage industries.

In an embodiment the variant of mannanase hydrolyses endo-beta-1,4-mannosidic linkages randomly.

In an embodiment the variant of mannanase, or the nucleotide sequence encoding the corresponding wild type mannanase, is obtainable or derivable from a bacterial source.

In an embodiment the variant of mannanase is fused with at least one further polypeptide, thus forming a fusion polypeptide. The fusion polypeptide or the further polypeptide may have other catalytic or binding activities in addition to those of mannanase. In an embodiment the further polypeptide comprises or consists of carbohydrate binding module, which is optionally a fragment of another protein or enzyme derived from the same or different organism as the mannanase.

In an embodiment the variant of mannanase is connected to the further polypeptide with a linker.

In an embodiment is provided a process for machine treatment of fabrics which process comprises treating fabric during a washing cycle of a machine washing process with a washing solution containing the variant of mannanase of the first aspect or the enzyme composition of the second aspect.

In an embodiment is provided a use of the enzyme composition of the second aspect or the variant of mannanase of the first aspect, together with an enzyme selected from protease, amylase, cellulase, lipase, xylanase, mannanase, cutinase, esterase, phytase, DNAse, pectinase, pectinolytic enzyme, pectate lyase, carbohydrase, arabinase, galactanase, xanthanase, xyloglucanase, laccase, peroxidase and oxidase with or without a mediator in a cleaning composition for fabric cleaning and/or fabric stain removal.

In an embodiment is provided a use of the variant of mannanase of the first aspect, or the enzyme composition of the second aspect, together with an enzyme selected from protease, amylase, cellulase, lipase, xylanase, mannanase, cutinase, esterase, phytase, DNAse, pectinase, pectinolytic enzyme, pectate lyase, carbohydrase, arabinase, galactanase, xanthanase, xyloglucanase, laccase, peroxidase and oxidase with or without a mediator in a cleaning composition for cleaning hard surfaces such as floors, walls, bathroom tile and the like.

In an embodiment is provided a use of the variant of mannanase of the first aspect, or the enzyme composition of the second aspect, together with an enzyme selected from protease, amylase, cellulase, lipase, xylanase, mannanase, cutinase, esterase, phytase, DNAse, pectinase, pectinolytic enzyme, pectate lyase, carbohydrase, arabinase, galactanase, xanthanase, xyloglucanase, laccase, peroxidase and oxidase with or without a mediator in a cleaning composition for hand and machine dishwashing.

### EXAMPLES

The following examples are provided to illustrate various aspects of the present invention. They are not intended to limit the invention, which is defined by the accompanying claims.

### Example 1. Variant design

To improve the stability and specific activity of the wild type Man7 mannanase, variant sets were designed based on structural analysis of wild type enzyme. The structural model of Man7 was created using Bioluminate software (Schrödinger LCC) with coordinates from *Bacillus* sp. Endo-Beta-D- 1,4-Mannanase (1WKY). The design included two or more specific mutations per variant. Table 1 shows list of variants. The amino acid numbering corresponds to the amino acid numbering of SEQ ID NO: 2 (Man7) full length amino acid sequence containing a signal sequence.

**Table 1. List of Man7 variants**

| Variant: | Mutations |
|---|---|
| TBH1: | M123I, S229A, G272Q, T285A |
| TBH2: | A158S, S229A, T285A, T307R |
| TBH3: | S229A, T285A, L316K |
| TBH4: | M123I, A158S, S229A, G272Q, T285A, T307R |
| TBH5: | M123I, S229A, G272Q, T285A, L316K |
| TBH6: | A158S, S229A, T285A, T307R, L316K |
| TBH7: | F180L, S229A, T285A, L316K |
| TBH8: | S229A, T285A |
| TBH9: | S229A, T285A, N300Q, N340Q, S400A, N419A, S433A, N446Q |
| TBH10: | A158S, S229A, T307R, L316K |
| TBH11: | A158S, T285A, T307R, L316K |

### Example 2. Cloning of synthetic mannanase variant genes

Standard molecular biology methods were used in the isolation and enzyme treatments of DNA (e.g. isolation of plasmid DNA, digestion of DNA to produce DNA fragments), in E. *coli* transformations, sequencing etc. The basic methods used were as described by the enzyme, reagent or kit manufacturer.

Variants *tbh1* - *tbh11* were ordered from GenScript as synthetic constructs without their own signal peptide encoding sequences and with codon optimization for *Trichoderma reesei.* Plasmid DNAs obtained from GenScript including the genes *tbh1* - *tbh11* were resuspended in sterile water, digested with *Nru*I and BamHI restriction enzymes (Thermo Fisher Scientific) according to manufacturer's instructions and cloned into an expression vector cleaved with *Nru*I and *Bam*HI. Ligation mixtures were transformed into *Escherichia coli* XL1-Blue or XL10-Gold cells (AH Diagnostics) and plated on LB (Luria-Bertani) plates containing 50-100 µg/ml ampicillin. Several E. *coli* colonies were collected from the plates and DNA was isolated with GenJet Plasmid Miniprep Kit (Thermo Fisher Scientific). Positive clones were screened using restriction digestions and they were shown to contain inserts of expected sizes. The fusion sites to the expression plasmid of *tbh1-tbh11* mannanase genes were sequenced and the plasmids were named pALK4415 - pALK4423, pALK4430 and pALK4431, respectively (For details see Example 4). The plasmid DNAs including the *tbh* genes delivered by GenScript were also transformed into XL10-Gold E. *coli* cells (Agilent) and deposited into DSMZ strain collection. The relevant information on the genes and the deduced amino acid sequences (SEQ ID NOs: 5-26) are summarized in Table 2 and Table 3, respectively. The E. *coli* strains RF12379 - RF12387, RF12456 and RF12457 including the plasmids pALK4434 - pALK4442, pALK4432 and pALK4433, respectively were deposited to the DSMZ collection under the accession numbers DSM 32425, DSM 32426, DSM 32427, DSM 32428, DSM 32429, DSM 32430, DSM 32431, DSM 32432, DSM32433, DSM 32518 and DSM 32519, respectively.

**Table 2. The summary on the mannanase variant encoding synthetic genes tbh1 - tbh11**

| **Gene** | **Length (bp)^{(a}** | **SEQ ID NO** |
|---|---|---|
| *tbh1* | 1395 | 5 |
| *tbh2* | 1395 | 7 |
| *tbh3* | 1395 | 9 |
| *tbh4* | 1395 | 11 |
| *tbh5* | 1395 | 13 |
| *tbh6* | 1395 | 15 |
| *tbh7* | 1395 | 17 |
| *tbh8* | 1395 | 19 |
| *tbh9* | 1395 | 21 |
| *tbh10* | 1395 | 23 |
| *tbh11* | 1395 | 25 |

| | | |
|---|---|---|
| ^{(a} The STOP codon is included | | |

**Table 3. The summary of the amino acid sequences deduced from the mannanase variant encoding gene sequences.**

| **Man protein** | **No of aas ^{(b}** | **Length of ss ^{(a}** | **CBM** | **Core (aa-aa)** | **Predicted (Da), ss not included ^{(b}** | **Predicted pl, ss not included ^{(b}** | **SEQ ID NO** |
|---|---|---|---|---|---|---|---|
| TBH1 | 464 | 26 | Yes | 27-331 | 50886 | 4.62 | 6 |
| TBH2 | 464 | 26 | Yes | 27-331 | 50904 | 4.67 | 8 |
| TBH3 | 464 | 26 | Yes | 27-331 | 50848 | 4.67 | 10 |
| TBH4 | 464 | 26 | Yes | 27-331 | 50957 | 4.67 | 12 |
| TBH5 | 464 | 26 | Yes | 27-331 | 50901 | 4.67 | 14 |
| TBH6 | 464 | 26 | Yes | 27-331 | 50919 | 4.72 | 16 |
| TBH7 | 464 | 26 | Yes | 27-331 | 50814 | 4.67 | 18 |
| TBH8 | 464 | 26 | Yes | 27-331 | 50833 | 4.62 | 20 |
| TBH9 | 464 | 26 | Yes | 27-331 | 50800 | 4.62 | 22 |
| TBH10 | 464 | 26 | Yes | 27-331 | 50949 | 4.72 | 24 |
| TBH11 | 464 | 26 | Yes | 27-331 | 50935 | 4.72 | 26 |

| | | | | | | | |
|---|---|---|---|---|---|---|---|
| ^{(a} The prediction on the signal sequence was made using the program SignalP v3.0, NN/HMM (Nielsen *et al.*, 1997; Nielsen & Krogh, 1998; Bendtsen *et al.*, 2004). ^{(b} The predicted signal sequence was not included. The prediction was made using Clone Manager Professional version 9 for Windows, Sci-Ed Software. | | | | | | | |

### Example 3. Production of recombinant mannanase variant proteins in Trichoderma reesei

Expression plasmids were constructed for production of recombinant mannanase TBH1 - TBH11 (SEQ ID NO: 6, 8, 10, 12, 14, 16, 18, 20, 22, 24 and 26) proteins in *Trichoderma reesei.* The expression plasmids constructed are listed in Table 4.

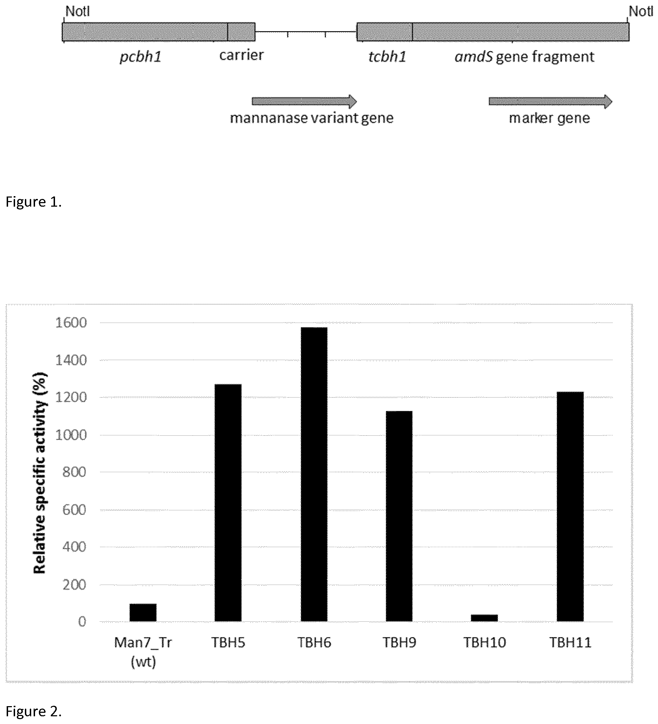

The recombinant mannanase genes without their own signal sequences were fused to the *T. reesei cel7A*/*cbh1* promoter with *T. reesei cel6A*/*cbh2* CBM carrier and linker followed by Kex2 protease recognition site. The transcription termination was ensured by the *T. reesei cel7A*/*cbh1* terminator and the *A. nidulans amd*S marker gene was used for selection of the transformants as described in Paloheimo *et al.* (2003). The linear expression cassettes (Fig. 1) were isolated from the vector backbones after *Not*I digestions and were transformed into *T. reesei* protoplasts. The host strains used, do not produce any of the four major *T. reesei* cellulases (CBHI, CBHII, EGI, EGII). The transformations were performed as in Penttilä *et al.* (1987) with the modifications described in Karhunen *et al.* (1993), selecting acetamidase as a sole nitrogen source (*amdS* marker gene). The transformants were purified on selection plates through single conidia prior to sporulating them on PD.

**Table 4. The expression cassettes constructed to produce TBH1 - TBH11 (SEQ ID NO: 6, 8, 10,1 2, 14, 16, 18, 20, 22, 24 and 26), recombinant proteins in Trichoderma reesei. The overall structure of the expression cassettes was as described in Fig. 1.**

| **Mannanase protein** | **Expression plasmid** | **Expression cassette ^{(a}** |
|---|---|---|
| TBH1 | pALK4415 | 7.5 kb *Not*I |
| TBH2 | pALK4416 | 7.5 kb *Not*I |
| TBH3 | pALK4417 | 7.5 kb *Not*I |
| TBH4 | pALK4418 | 7.5 kb *Not*I |
| TBH5 | pALK4419 | 7.5 kb *Not*I |
| TBH6 | pALK4420 | 7.5 kb *Not*I |
| TBH7 | pALK4421 | 7.5 kb *Not*I |
| TBH8 | pALK4422 | 7.5 kb *Not*I |
| TBH9 | pALK4423 | 7.5 kb *Not*I |
| TBH10 | pALK4430 | 7.5 kb *Not*I |
| TBH11 | pALK4431 | 7.5 kb *Not*I |

| | | |
|---|---|---|
| ^{(a} The expression cassette for *T. reesei* transformation was isolated from vector backbone by using *Not*I digestions. | | |

The mannanase production of the transformants was analyzed from the culture supernatants of the shake flask cultivations. The transformants were inoculated from the PD slants to shake flasks containing 50 ml of complex lactose-based cellulase inducing medium (Joutsjoki *at al.* 1993) buffered with 5% KH₂PO₄. The mannanase protein production of the transformants was analyzed from culture supernatants after growing them for 7 days at 30 °C, 250 rpm Heterologous production of recombinant proteins was analyzed by SDS-PAGE with subsequent Coomassie staining. The supernatants were recovered also for application tests by centrifugation.

### Example 4. Assay of galactomannanase activity by DNS -method

Mannanase activity (MNU) was measured as the release of reducing sugars from galactomannan (0.3 w/w-%) at 50°C and pH 7.0 in 5 min. The amount of released reducing carbohydrates was determined spectrophotometrically using dinitrosalicylic acid.

Substrate (0,3 w/w-%) used in the assay was prepared as follows: 0.6 g of locust bean gum (Sigma G-0753) was in 50 mM sodium citrate buffer pH 7 (or citrate phosphate buffer pH 7) at about 80 °C using a heating magnetic stirrer and heated up to boiling point. The solution was cooled and let to dissolve overnight in a cold room (2 - 8 °C) with continuous stirring and insoluble residues were removed by centrifugation. After that solution was filled up to 200 ml by buffer. Substrate was stored as frozen and melted by heating in a boiling water bath to about 80 °C, cooled to room temperature and mixed carefully before use.

DNS reagent used in the assay was prepared by dissolving 50 g of 3.5-dinitrosalisylic acid (Sigma D-550) in about 4 liter of water. With continuous magnetic stirring 80.0 g of NaOH was gradually added and let to dissolve. An amount of 1500 g of Rochelle Salt (K-Na-tartrate, Merck 8087) was added in small portions with continuous stirring. The solution that was cautiously warmed to a maximum temperature of 45 °C, was cooled to room temperature and filled up to 5000 ml. After that it was filtered through Whatman 1 filter paper and stored in a dark bottle at room temperature.

The reaction was first started by adding 1.8 ml of substrate solution to each of the two test tubes and let to equilibrate at 50 °C for 5 minutes, after which 200 µl of suitably diluted enzyme solution was added to one of the tubes, mixed well with vortex mixer and incubated exactly for 5 min at 50 °C. Enzyme blanks didn't need to be equilibrated or incubated. The reaction was stopped by adding 3.0 ml of DNS reagent into both tubes and mixed. 200 µl of sample solution was added to the enzyme blank tubes. Both tubes were placed in a boiling water bath. After boiling for exactly 5 minutes, the tubes were placed in a cooling water bath and allow them to cool to room temperature. The absorbance of sample was measured against the enzyme blank at 540 nm and activity was read from the calibration curve and multiplied by the dilution factor. A suitable diluted sample yielded an absorbance difference of 0.15 - 0.4.

Standard curve was prepared 20 mM from mannose stock solution by dissolving 360 mg of mannose (SigmaM-6020, stored in a desiccator) in assay buffer and diluted to solutions containing 3, 6, 10 and 14 µmol/ml of mannose. Standards were handled like the samples except for incubating at 50°C. The absorbances were measured against the reagent blank (containing buffer instead of standard dilution of mannose) at 540 nm. Calibration curve was constructed for every series of assays.

One mannanase unit (MNU) was defined as the amount of enzyme that produces reductive carbohydrates having a reductive power corresponding to one nmol of mannose from galactomannan in one second under the assay conditions (1 MNU = 1nkat).

### Example 5. Specific activities of the purified mannanase variants

Cells and solids were removed from the fermentation culture medium by centrifugation for 10 min, 4000 g at 4°C. The supernatant of 10 ml was used for protein purification. The sample was filtered through 0.44 µm PVDF membrane (Millex-HV, Merck Millipore Ltd, Carrigtwohill, IRL). The filtrate was loaded onto a HiPrep 26/10 Desalting column (GE Healthcare, Uppsala, Sweden) equilibrated in 20 mM HEPES pH 7. The desalted sample was then loaded onto a 5 ml HiTrap Q HP column (GE Healthcare, Uppsala, Sweden) pre-equilibrated with 20 mM HEPES pH 7. After sample loading, the column was washed with the same buffer for 20 ml. Proteins were eluted with linear salt gradient 20 mM HEPES, 500 mM NaCl pH 7 in 15 CVs. Fractions of 5 ml were collected and analyzed on SDS-PAGE. The fractions containing target protein were combined and concentrated to 2 ml using Vivaspin 20, 10 kDa MWCO ultrafiltration devices (GE Healthcare). The concentrated sample was further fractionated using Superdex 75 26/60 gelfiltration column equilibrated with 20 mM MES, 200 mM NaCl pH 6,5. Fractions of 2 ml were collected and analyzed by SDS-PAGE. Fractions containing pure mannanase were combined.

Purified samples were above 95% pure.

Enzyme content of the purified sample was determined using UV absorbance 280 nm measurements. Excitation coefficients for each mannanases were calculated on the bases of amino acid sequence of the enzyme by using ExPASy (Server http://web.expasy.org/protparam/). (Gasteiger *et al.* 2005).

The enzyme activity (MNU) of purified samples was measured as release of reducing sugars as described in Example 4.

The specific activity (MNU/mg) of mannanases was calculated by dividing MNU activity of purified sample with the amount of purified enzyme. Relative specific activity (%) was calculated by dividing specific activity of variant mannanase by specific activity of wild type Man7 mannanase.

According to 3D model of wild type Man7 potential glycosylation site asparagine 283 locates near the catalytic center of enzyme. Glycosylation of asparagine 283 could lead to the reduced catalytic activity of enzyme. To improve the specific activity of mannanase threonine 285 was mutated to alanine. This mutation prevents N-glycosylation of asparagine 283.

The results indicated that the specific activity of the wild type Man7 enzyme produced in *Trichoderma* was increased more than 10 fold when T285 was mutated to alanine (TBH5, TBH6, TBH9 and TBH11) (Fig. 2). On the other hand, specific activity of the TBH10 variant, in which glycosylation sites other than N283 were eliminated, was not improved (Fig. 2).

The results clearly indicated that the mutation which prevent glycosylation of N283 improves the specific activity of mannanase variant while eliminating other glycosylation sites do not show significant effects on specific activity. Production yield (measured as mannanase activity) was higher with TBH5, TBH6, TBH9 and TBH11 (data not shown) compared to wild type Man7.

Mannanase variants with higher specific activity result to the decreased production costs and therefore are commercially more feasible.

### Example 6. Stain removal performance of mannanase variants produced in Trichoderma with commercial detergents

Cultivation supernatants of TBH1 -TBH11 variants produced in *Trichoderma* (as described in Example 3) were tested for their ability to remove mannanase sensitive standard stains at 40 °C and water hardness of 16°dH with commercial detergents and compared to wild type Man7 enzyme. The following artificially soiled test cloths from Center for testmaterial B.V. (the Netherlands) were used: Chocolate pudding mannanase sensitive on cotton (E-165), Locust bean gum, with pigment on cotton (C-S-73) and Guar gum with carbon black on cotton (C-S-43). The fabric was cut in 6 cm x 6 cm swatches and 2 pieces of each were used in test.

Commercial heavy duty liquid detergent A containing all other enzymes except mannanase was used at concentration of 4.4 g per liter of wash liquor, Commercial Color detergent powder without enzymes was used at 3.8 g/l and Commercial bleach detergent powder without enzymes was used at 4.2 g/l. Detergent containing wash liquors we prepared in synthetic tap water with hardness of 16 °dH. Protease Savinase® 16 L (0.5 w/w %) and amylase Stainzyme® 12 L (0.4 w/w %) was added into hard water used with commercial color and bleach detergent powders, the liquid detergent already contained amylase and protease. pH of the wash liquor of liquid detergent was approximately 8.3, with color detergent powder approx. 10 and with the bleach detergent approx. 9.5.

Mannanases were dosed as 0.025 and/or 0.05 MNU activity per ml of wash liquor. Activity was measured as described in Example 4. Control sample contained the detergent solution but no mannanase.

For synthetic tap water with hardness of 16 °dH the following stock solutions were prepared in deionized water (Milli-Q or equivalent):
Stock solution with 1000 °d Calcium-hardness: CaCl2 x 2 H2O (1.02382.1000, Merck KGaA, Germany) 26,22 g/l
Stock solution with 200 °d Magnesium-hardness: MgSO4 x 7 H2O (1.05886.1000, Merck KGaA, Germany) 8,79 g/l H2O
NaHCO3 stock solution: NaHCO3 (1.06329.1000 Merck KGaA, Germany) 29,6 g/l 13,3 ml CaCl2 solution, 13,3 ml MgSO4 solution and 10,0 ml of freshly made NaHCO3 solution were added in volumetric flask in the given order, made up to 1 liter with deionized water and mixed. The hardness of water was determined by complexometric titration and found correct.

Stain removal treatments were performed in Atlas LP-2 Launder-Ometer as follows. Launder-Ometer was first preheated to 40°C. Then detergent, 250 ml synthetic tap water with hardness of 16 °dH and diluted enzyme (<1,0 ml) were added into 1,2 liter containers. Stains were added and the Launder-Ometer was run at 40°C for 60 min with a rotation speed of 42 rpm. After that the swatches were carefully rinsed under running water and dried overnight at indoor air, on a grid protected against daylight.

The stain removal effect was evaluated by measuring the colour as reflectance values with Konica Minolta CM-3610A spectrophotometer using L*a*b* color space coordinates (illuminant D65/10°, 420 nm cut). Fading of the stains, indicating mannanase performance (stain removal efficiency) was calculated as ΔL* (delta L*), which means lightness value L* of enzyme treated fabric minus lightness value L* of fabric treated with washing liquor without mannanase (control). Final results (total stain removal effect) were shown as sum of ΔL* of each stains. Color values of each stains were average of 2 swatches.

The results obtained with commercial liquid detergent are shown in Figs 3 A and B, the results with commercial color detergent powder in Figs 4 A and B and the results with commercial bleach detergent in Fig 5. All variants (TBH1-TBH11) showed excellent stain removal performance with commercial detergents of different types. Performance of variants was similar to wild type.

### Example 7. Efficiency study with mannanase alone and in combination with a non-starch polysaccharide (NSP) degrading enzyme in broilers

Effects of recombinant mannanase variants of the invention are studied on growth in broilers. Ultrafiltrate of the fermentation broth including a recombinant mannanase variant is dried and target levels applied to a pelleted broiler diet alone or in combination with a commercial available xylanase based product.

A control diet based on corn and dehulled solvent extracted soybean meal is fed without enzyme or added by different levels of the recombinant mannanase variants of the invention alone or in combination with a standard dose of a commercial xylanase.

Initial weight of the broilers is between 30 g and 50 g. The trial lasts between three and five weeks. Each treatment consists at minimum of six replicates with 10 broilers each. In each case the diet is analysed for moisture, crude protein, crude fibre, fat, ash, and enzyme protein.

Five diets are prepared:
1) unsupplemented control (BD)
2) BD + mannanase 1 - 500 mg/kg
3) BD + mannanase 1 - 1000 mg/kg
4) BD + mannanase 1 - 500 mg/kg + xylanase 1 - 10 mg/kg
5) BD + xylanase 1 - 10 mg/kg

Health status and mortality of the animals is checked daily by visual inspection. At days 0, 14, and 35 body weight gain (BW), feed intake (FI), and feed-conversion ratio (FCR) are measured. FCR is calculated as the total feed consumed divided by the weight gain during the same period. Determination of the effect of the recombinant mannanase variants is based on the comparison to those animals fed the same diet or the same diet but added by xylanase.

### Example 8. Instant coffee production

Pure mannan is the main storage polysaccharide component of coffee endosperms and is responsible for their high viscosity, which negatively affects the technological processing of instant coffee and increases energy consumption during drying. Those effects are attributed to mannan forming hard, insoluble crystalline structures. β-mannanase, often together with other enzymes such as pectinase and cellulase, is added during the concentration step of instant coffee production to reduce viscosity in coffee extracts. Mannanase is also be employed for hydrolyzing galactomannans present in a liquid coffee extract in order to inhibit gel formation during freeze drying of instant coffee. Furthermore, due to the use of enzymatic treatment the coffee bean extracts can be concentrated by a low cost procedure such as evaporation.

The test is performed according the following flow-chart of Fig. 6 at temperatures of 10°C and an enzyme dosage of 0.15% d.s.

Mannanase variants of the invention are tested in mixture composed of different enzymes, such as pectinases and cellulases.

The viscosity of the coffee extract increases significantly over time under standard process conditions. However, the viscosity is significantly reduced using the enzyme mixture containing the mannanase variants of the invention resulting an improved downstream processing such as spray- or freeze drying.

### Example 9. Pineapple processing

In particular, mannanase is useful for pineapple mill juice extraction and clarification, as pineapple contains a large fraction of mannans, including glucomannans and galactomannans.

Mannanase helps to improve extraction of valuable fruit components, lower the viscosity of fruit juice prior to concentration, and increase filtration rate and stability of the final product.

The pineapples are crushed in a meat grinder and fill 500 g mash in a 1000 ml beaker. The enzyme is applied at 21 °C with a reaction time of 60 minutes. The mash is then pressed with a small Hafico press according to the press protocol: 0 bar 2 min - 50 bar 2 min - 100 bar 2 min - 150 bar 2 min - 200 bar 1 min - 300 bar 1 min - 400 bar 1min. The obtained juice is then centrifuged at 4500 rpm for 5 minutes and analyzed for turbidity and viscosity.

Mannanase variants of the invention are tested in enzyme mixtures A, B and C (Table 5).

The enzymes are first diluted with tab water before being added to the pineapple mash.

**Table 5. Enzyme mixtures**

| | **Enzyme activity** | **Dosage [ppm]** | **5 ml of [% enzyme solution]** |
|---|---|---|---|
| blank | | | 5 ml H2O |
| Mixture A | Pectinase | 50 | 0.50% |
| Mixture B | Pectinase + Arabanase | 50 | 0.50% |
| Mixture C | Pectinase + Mannanase | 50 | 0.50% |

Applying mannanase variants of the invention leads to increased yield and lower turbidity of juice in pineapple processing.

### Example 10. Mannanase treatment of soya beans for soya milk production

For the enzymatic treatment of soya beans to get soya milk the "hot process" is commonly used. For the hot soya milk process the dried soya beans were mixed and crushed in a mixer with boiling tap water in a ratio of 1:7 (soaked beans: water). The whole soya slurry is cooled down to 50-55 °C before enzyme addition. The pH level for the soya slurry should be around pH 6.5 and can be adjusted with NaHCO₃. The mannanase enzyme is dosed at 1 kg/t of dried soya beans into the slurry and stirred for 30 min. After completion of the reaction time, the slurry is pressed using a laboratory press to obtain the final product: soya milk. In order to ensure the same pressing profile, the pressure as well as the corresponding pressing time is specified, as shown in Table 6. Besides the sample for enzymatic reaction, a control sample without any enzyme is prepared, in which the enzyme solution was replaced with water.

**Table 6. Press scheme**

| | | | | |
|---|---|---|---|---|
| **pressure [bar]** | 0 | 50 | 100 | 300 |
| **time [min]** | 2 | 2 | 2 | 1 |

After pressing the soya milk is heated in a microwave until boiling to stop the enzyme reaction. Analysis of the soya milk:
- Yield in gram/time
- °Brix, which gives a direct correlation of the amount of sugar in the soy milk, is determined with a refractometer
- The turbidity of the juice is measured with a NTU-photometer, which measures the nephelometric turbidity.
- The brightness will be measured with a LAB-measurement
- Protein content is determined with a CN-Analyser (combustion method)
- Flavour

Soya milk treated with the mannanase variants of the invention shows a increased yield, brighter colour, increased °Brix, a lower turbidity, a higher protein content and a better taste (off flavour removal).

Without limiting the scope and interpretation of the patent claims, certain technical effects of one or more of the aspects or embodiments disclosed herein are listed in the following: A technical effect is degradation or modification of mannan. Another technical effect is provision of mannanase which has good storage stability.

The foregoing description has provided by way of non-limiting examples of particular implementations and embodiments of the invention a full and informative description of the best mode presently contemplated by the inventors for carrying out the invention. It is however clear to a person skilled in the art that the invention is not restricted to details of the embodiments presented above, but that it can be implemented in other embodiments using equivalent means without deviating from the characteristics of the invention.

Furthermore, some of the features of the above-disclosed aspects and embodiments of this invention may be used to advantage without the corresponding use of other features. As such, the foregoing description should be considered as merely illustrative of the principles of the present invention, and not in limitation thereof. Hence, the scope of the invention is only restricted by the appended patent claims.

In an embodiment at least one component of the compositions of the invention has a different chemical, structural or physical characteristic compared to the corresponding natural component from which the at least one component is derived from. In an embodiment said characteristic is at least one of uniform size, homogeneous dispersion, different isoform, different codon degeneracy, different post-translational modification, different methylation, different tertiary or quaternary structure, different enzyme activity, different affinity, different binding activity, and different immunogenicity.

### References

Bendtsen JD, Nielsen H, von Heijne G, and Brunak S. (2004) Improved prediction of signal peptides: SignalP 3.0. J. Mol.Biol. 340:783-795.
Gasteiger E., Hoogland C., Gattiker A., Duvaud S., Wilkins M.R., Appel R.D., Bairoch A.;Protein Identification and Analysis Tools on the ExPASy Server; (In) John M. Walker (ed): The Proteomics Protocols Handbook, Humana Press (2005). pp. 571-607.
Joutsjoki W, Torkkeli TK, and Nevalainen KMH. (1993) Transformation of Trichoderma reesei with the Hormoconis resinae glucoamylase P (gamP) gene: production of a heterologous glucoamylase by Trichoderma reesei. Curr. Genet. 24: 223-228.
Karhunen T, Mäntylä M, Nevalainen KMH, and Suominen PL. (1993) High frequency one-step gene replacement in Trichoderma reesei. I. Endoglucanase I overproduction. Mol. Gen. Genet. 241: 515-522.
Nielsen H, Engelbrecht J, Brunak S, and von Heijne G. (1997) Identification of prokaryotic and eykaryotic signal peptides and prediction of their cleavage sites. Protein. Eng. 10: 1-6.
Nielsen H, and Krogh A. (1998) Prediction of signal peptides and signal anchors by a hidden Markov model. In: Proceedings of the Sixth International Conference on Intelligent Systems for Molecular Biology (ISMB 6), AAAI Press, Menlo Park, California, p. 122-130.
Paloheimo M, Mäntylä A, Kallio J, and Suominen P. (2003) Highyield production of a bacterial xylanase in the filamentous fungus Trichoderma reesei requires a carrier polypeptide with an intact domain structure. Appl. Env. Microbiol. 69: 7073-7082.
Penttilä M, Nevalainen H, Rättö M, Salminen E, and Knowles J. (1987) A versatile transformation system for the cellulolytic filamentous fungus Trichoderma reesei. Gene 61: 155-164.

## Claims

1. A variant of mannanase having mannanase activity and a non-glycosylated amino acid at the position 283, wherein the variant is selected from the group consisting of
1) a polypeptide having at least 85% sequence identity to residues 27-331 of SEQ ID NO: 2;
2) a polypeptide encoded by a polynucleotide that hybridizes under high stringency conditions with
a) nucleotides 79 - 993 of SEQ ID NO: 1 (*man7*) or
b) the full-length complement of a),
and wherein the high stringency conditions correspond to hybridizing at prehybridisation and hybridization at 65°C in 5× SSC, 0.1% N-lauroylsarcosine, 0.02% SDS, 1% blocking reagent (Roche 11 096 176 001), following standard Southern blotting procedures for 12 to 24 hours, and washing two to three times for 15 minutes using 0.1X SSC, 0.1% SDS at 65°C.; and
3) a variant encoded by a polynucleotide having at least 95% sequence identity to the SEQ ID NO: 1 or the genomic DNA sequence thereof;
and wherein the amino acid numbering corresponds to the amino acid numbering of SEQ ID NO: 2 (Man7) full length amino acid sequence containing a signal sequence, and wherein at least position N283, T285, or S285 is substituted by a residue which prevents N-linked glycosylation of the residue 283 when expressed in a host cell capable of N-linked glycosylation.

2. The variant of any one of claim 1, wherein the position T285 or S285 is substituted to a residue other than T or S.

3. An enzyme composition comprising the variant of mannanase of any one of claims 1-2 and
a. at least one preservative selected for example from group consisting of organic acid, citric acid, ascorbic acid, benzoic acid and their salts and derivatives, sodium benzoate, benzoate hydroxybenzoate and derivatives, sorbic acid, sodium sorbate, sorbate, salts, such as sodium chloride or potassium chloride, 1,2-Benzisothiazolin-3-one (BIT), or a combination thereof;
b. optionally at least one stabilizer selected from polyol, propylene glycol, polyethylene glycol, hexylene glycol, glycerol, a sugar, sugar alcohol, polysaccharide, lactic acid, boric acid, boric acid derivative, aromatic borate ester, 4-formylphenyl boronic acid, phenyl boronic acid derivative, peptide, surfactant, or a combination thereof;
c. optionally at least one enzyme selected from proteases, amylases, cellulases, lipases, xylanases, mannanases, cutinases, esterases, phytases, DNAses, pectinases, pectinolytic enzymes, pectate lyases, carbohydrases, arabinases, galactanases, xanthanases, xyloglucanases, laccases, peroxidases and oxidases with or without a mediator, or a combination thereof; and
d. optionally at least one filler selected from maltodextrin, flour, sodium chloride, sulfate, sodium sulfate, or a combination thereof.

4. The enzyme composition of claim 3 in the form of a liquid composition or a solid composition such as solution, dispersion, paste, powder, granule, granulate, coated granulate, tablet, cake, crystal, crystal slurry, gel, or pellet.

5. A detergent composition comprising the variant of mannanase of any one of claims 1-2 or the enzyme composition of claims 3-4.

6. The detergent composition of claim 5 in a form of a liquid detergent or a solid detergent preferably in a form of a bar, a homogenous tablet, a tablet having two or more layers, a pouch having one or more compartments, a regular or compact powder, a granule, a granulate, a paste, a gel, or a regular, compact or concentrated liquid.

7. The detergent composition of claim 5 or 6 further comprising one or more additional enzyme selected from the group consisting of protease, lipase, cutinase, amylase, carbohydrase, cellulase, pectinase, pectatelyase, pectinolytic enzyme, esterase, mannanase, arabinase, galactanase, xylanase, oxidase, xanthanase, xyloglucanase, laccase, DNAse and/or peroxidase, preferably selected from the group consisting of proteases, amylases, cellulases and lipases.

8. A recombinant host cell comprising genetic elements that allow producing at least recombinant polypeptide comprising the variant of mannanase of claims 1-2, and wherein the genetic elements comprise at least one control sequence which controls the production of the recombinant polypeptide in the recombinant host cell.

9. The recombinant host cell of 8, wherein the host cell is selected from the group consisting of:
fungal cells,
filamentous fungal cells from Division *Ascomycota,* Subdivision *Pezizomycotina*; preferably from the group consisting of members of the Class *Sordariomycetes,* Subclass *Hypocreomycetidae,* Orders *Hypocreales* and *Microascales* and *Aspergillus, Chrysosporium, Myceliophthora* and *Humicola;*
more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium*;
more preferably from the group consisting of *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,*
bacterial cells, preferably gram positive Bacilli such as *B. subtilis, B. licheniformis, B. megaterium, B. amyloliquefaciens, B. pumilus,* gram negative bacteria such as *Escherichia coli,* actinomycetales such as *Streptomyces* sp., and
yeasts, such as *Saccharomyces cerevisiae, Pichia pastoris, Yarrowia lipolytica,*
most preferably *Trichoderma reesei.*

10. The recombinant host cell of any one of claims 8-9, wherein the recombinant polypeptide is a fusion protein, which further comprises at least one of:
an amino acid sequence providing a secretory signal sequence;
an amino acid sequence which facilitates purification, such as an affinity tag or His-tag;
an amino acid sequence which enhances production, such as an amino acid sequence which is a carrier, such as CBM;
an amino acid sequence having an enzyme activity; and
an amino acid sequence providing for the fusion protein with binding affinity, such as a carbohydrate binding moiety.

11. A method for producing a recombinant polypeptide having mannanase activity comprising:
a. cultivating a recombinant host cell of any one of claims 8-10, wherein the genetic elements comprise at least one control sequence which controls the production of the recombinant polypeptide in the recombinant host cell; the genetic elements optionally comprise at least one sequence encoding a signal sequence for transporting the recombinant polypeptide outside the host cell; and cultivating is carried out in conditions allowing production of the recombinant polypeptide; and
a. recovering the recombinant polypeptide.

12. A method for degrading or modifying mannan containing material comprising treating said mannan containing material with an effective amount of the enzyme composition of any one of claims 3-4 or the variant of any one of claims 1-2.

13. The method of claim 12 wherein the mannan containing material is plant based material, textile, waste water, sewage, oil, or a combination thereof.

14. An animal feed comprising the enzyme composition of any one of claims 3-4 or the variant of mannanase of any one of claims 1-2, and at least one protein source of plant origin or a mannan containing product or by-product, and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

15. A feed supplement comprising the enzyme composition of any one of claims 3-4 or the variant of mannanase of any one of claims 1-2; and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate or a combination thereof.

16. A use of the variant of claims 1-2 or the enzyme composition of claims 3-4 in oil drilling or hydro-fracturing.

17. A use of the variant of claims 1-2 or the enzyme composition of claims 3-4 in processing coffee extract, fruit juice, pineapple juice, or soya milk.

## Patentansprüche

1. Variante von Mannanase mit Mannanase-Aktivität und einer nicht-glykosylierten Aminosäure an der Position 283, wobei die Variante ausgewählt ist aus der Gruppe bestehend aus
1) einem Polypeptid mit wenigstens 85 % Sequenzidentität zu Rückständen 27 - 331 von SEQ ID NO: 2;
2) ein Polypeptid, codiert durch ein Polynucleotid, das hybridisiert unter Bedingungen hoher Stringenz mit
a) Nukleotiden 79 - 993 von SEQ ID NO: 1 (man7) oder
b) dem Komplement voller Länge von a),
und wobei die Bedingungen hoher Stringenz entsprechen dem Hybridisieren bei Prähybridisierung und Hybridisierung bei 65 °C in 5 × SSC, 0,1 % N-Lauroylsarcosin, 0,02 % SDS, 1 % Blockierungsreagenz (Roche 11 096 176 001), folgend standardmäßiges Southern-Blotting-Verfahren für 12 bis 24 Stunden, und Waschen zwei- bis dreimal für 15 Minuten unter Verwendung von 0,1 × SSC, 0,1 % SDS bei 65 °C; und
3) eine Variante, codiert durch ein Polynucleotid mit wenigstens 95 % Sequenzidentität zu der SEQ ID NO: 1 oder der genomischen DNA-Sequenz davon;
und wobei die Aminosäure-Nummerierung der Aminosäure-Nummerierung von SEQ ID NO: 2 (Man7) Aminosäuresequenz voller Länge entspricht, die eine Signalsequenz enthält, und wobei wenigstens Position N283, T285 oder S285 substituiert ist durch einen Rückstand, der N-verknüpfte Glykosylierung des Rückstandes 283 verhindert wenn exprimiert in einer Wirtszelle, die zu N-verknüpfter Glykosylierung fähig ist.

2. Variante nach Anspruch 1, wobei die Position T285 oder S285 substituiert ist in einen Rückstand anders als T oder S.

3. Enzymzusammensetzung, umfassend die Variante von Mannanase nach einem der Ansprüche 1 - 2 und
a. wenigstens einen Konservierungsstoff, ausgewählt beispielsweise aus Gruppe bestehend aus organischer Säure, Zitronensäure, Ascorbinsäure, Benzoesäure und ihren Salzen und Derivaten, Natriumbenzoat, Benzoat Hydroxybenzoat und Derivaten, Sorbinsäure, Natriumsorbat, Sorbat, Salze, wie z.B. Natriumchlorid oder Kaliumchlorid, 1,2-Benzisothiazolin-3-on (BIT), oder eine Kombination davon;
b. optional wenigstens einen Stabilisator, ausgewählt aus Polyol, Propylenglykol, Polyethylenglykol, Hexylenglykol, Glycerin, einem Zucker, Zuckeralkohol, Polysaccharid, Milchsäure, Borsäure, Borsäurederivat, aromatischem Boratester, 4-Formylphenylboronsäure, Phenylboronsäurederivat, Peptid, Tensid, oder eine Kombination davon;
c. optional wenigstens ein Enzym, ausgewählt aus Proteasen, Amylasen, Cellulasen, Lipasen, Xylanasen, Mannanasen, Cutinasen, Esterasen, Phytasen, DNAsen, Pektinasen, pektinolytischen Enzymen, Pektatlyasen, Carbohydrasen, Arabinasen, Galactanasen, Xanthanasen, Xyloglucanasen, Laccasen, Peroxidasen und Oxidasen mit oder ohne einem Botenstoff, oder einer Kombination davon; und
d. optional wenigstens einen Füllstoff, ausgewählt aus Maltodextrin, Mehl, Natriumchlorid, Sulfat, Natriumsulfat oder einer Kombination davon.

4. Enzymzusammensetzung nach Anspruch 3 in der Form einer flüssigen Zusammensetzung oder einer festen Zusammensetzung wie z.B. Lösung, Dispersion, Paste, Pulver, Korn, Granulat, beschichtetes Granulat, Tablette, Kuchen, Kristall, Kristallaufschlämmung, Gel oder Pellet.

5. Detergenszusammensetzung, umfassend die Variante von Mannanase nach einem der Ansprüche 1 - 2 oder die Enzymzusammensetzung der Ansprüche 3 - 4.

6. Detergenszusammensetzung nach Anspruch 5 in einer Form eines flüssigen Detergens oder eines festen Detergens, bevorzugt in einer Form eines Riegels, einer homogenen Tablette, einer Tablette mit zwei oder mehr Schichten, eines Beutels mit einer oder mehreren Kammern, eines regulären oder kompakten Pulvers, eines Korns, eines Granulats, einer Paste, eines Gels oder einer normalen, kompakten oder konzentrierten Flüssigkeit.

7. Detergenszusammensetzung nach Anspruch 5 oder 6, ferner umfassend ein oder mehrere zusätzliche Enzyme, ausgewählt aus der Gruppe bestehend aus Protease, Lipase, Cutinase, Amylase, Carbohydrase, Cellulase, Pektinase, Pectatelyase, pektinolytischem Enzym, Esterase, Mannanase, Arabinase, Galactanase, Xylanase, Oxidase, Xanthanase, Xyloglucanase, Laccase, DNAse und / oder Peroxidase, bevorzugt ausgewählt aus der Gruppe bestehend aus Proteasen, Amylasen, Cellulasen und Lipasen.

8. Rekombinante Wirtszelle, umfassend genetische Elemente, die Herstellen von wenigstens rekombinantem Polypeptid ermöglichen, umfassend die Variante von Mannanase nach Ansprüchen 1 - 2, und wobei die genetischen Elemente umfassen wenigstens eine Steuersequenz, die die Herstellung des rekombinanten Polypeptids in der rekombinanten Wirtszelle steuert.

9. Rekombinante Wirtszelle nach 8, wobei die Wirtszelle ausgewählt ist aus der Gruppe bestehend aus:
Pilzzellen,
filamentösen Pilzzellen der Abteilung Ascomycota, Unterabteilung Pezizomycotina; bervorzugt aus der Gruppe bestehend aus Mitgliedern der Klasse Sordariomycetes, Unterklasse Hypocreomycetidae, Ordnungen Hypocreales und Microascales und Aspergillus, Chrysosporium, Myceliophthora und Humicola;
besonders bevorzugt aus der Gruppe bestehend aus Familien Hypocreacea, Nectriaceae, Clavicipitaceae, Microascaceae und Gattungen Trichoderma (Anamorph von Hypocrea), Fusarium, Gibberella, Nectria, Stachybotrys, Claviceps, Metarhizium, Villosiclava, Ophiocordyceps, Cephalosporium und Scedosporium;
besonders bevorzugt aus der Gruppe bestehend aus Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum, und Scedosporium apiospermum, und Aspergillus niger, Aspergillus awamori, Aspergillus oryzae, Chrysosporium lucknowense, Myceliophthora thermophila, Humicola insolens und Humicola grisea, bakteriellen Zellen, bevorzugt grampositive Bazillen wie z.B. B. subtilis, B. licheniformis, B. megaterium, B. amyloliquefaciens, B. pumilus, gramnegative Bakterien wie z.B. Escherichia coli, Actinomycetales wie z.B. Streptomyces sp., und
Hefen wie z.B. Saccharomyces cerevisiae, Pichia pastoris, Yarrowia lipolytica, am bevorzugtesten Trichoderma reesei.

10. Rekombinante Wirtszelle nach einem der Ansprüche 8 - 9, wobei das rekombinante Polypeptid ein Fusionsprotein ist, das ferner umfasst wenigstens eines von:
eine Aminosäuresequenz, die eine sekretorische Signalsequenz bereitstellt;
eine Aminosäuresequenz, die Reinigung erleichtert, wie z.B. eine Affinitätsmarkierung oder eine His-Markierung;
eine Aminosäuresequenz, die Herstellung verbessert, wie z.B. eine Aminosäuresequenz, die ein Träger ist, wie z.B. CBM;
eine Aminosäuresequenz mit einer Enzymaktivität; und
eine Aminosäuresequenz, die das Fusionsprotein mit Bindungsaffinität bereitstellt, wie z.B. ein Carbohydrat-Bindungsteil.

11. Verfahren zum Herstellen eines rekombinanten Polypeptids mit Mannanase-Aktivität, umfassend:
a. Kultivieren einer rekombinanten Wirtszelle nach einem der Ansprüche 8 - 10, wobei
die genetischen Elemente wenigstens eine Steuersequenz umfassen, die die Herstellung des rekombinanten Polypeptids in der rekombinanten Wirtszelle steuert;
die genetischen Elemente optional wenigstens eine Sequenz umfassen, die eine Signalsequenz codiert zum Transportieren des rekombinanten Polypeptids außerhalb der Wirtszelle; und
Kultivieren durchgeführt wird unter Bedingungen, die Produktion des rekombinanten Polypeptids ermöglichen; und
a. Gewinnung des rekombinanten Polypeptids.

12. Verfahren zum Abbauen oder Modifizieren von Mannan enthaltendem Material, umfassend Behandeln des Mannan enthaltenden Materials mit einer wirksamen Menge der Enzymzusammensetzung nach einem der Ansprüche 3 - 4 oder der Variante nach einem der Ansprüche 1 - 2.

13. Verfahren nach Anspruch 12, wobei das Mannan enthaltende Material pflanzliches Material, Gewebe, Abwasser, Schmutzwasser, Öl, oder eine Kombination davon ist.

14. Tierfutter, umfassend die Enzymzusammensetzung nach einem der Ansprüche 3 - 4 oder die Variante von Mannanase nach einem der Ansprüche 1 - 2, und wenigstens eine Proteinquelle pflanzlichen Ursprungs oder ein Mannan enthaltendes Produkt oder Beiprodukt, und
a. optional wenigstens ein Enzym, ausgewählt aus Protease, Amylase, Phytase, Xylanase, Endoglucanase, Beta-Glucanase oder einer Kombination davon; und
b. optional wenigstens ein Füllstoff, ausgewählt aus Maltodextrin, Mehl, Salz, Natriumchlorid, Sulfat, Natriumsulfat oder einer Kombination davon.

15. Futterzusatz, umfassend die Enzymzusammensetzung nach einem der Ansprüche 3 - 4 oder die Variante von Mannanase nach einem der Ansprüche 1 - 2; und
a. optional wenigstens ein Enzym, ausgewählt aus Protease, Amylase, Phytase, Xylanase, Endoglucanase, Beta-Glucanase oder einer Kombination davon; und
b. optional wenigstens ein Füllstoff, ausgewählt aus Maltodextrin, Mehl, Salz, Natriumchlorid, Sulfat, Natriumsulfat oder einer Kombination davon.

16. Verwendung der Variante nach Ansprüchen 1 - 2 oder der Enzymzusammensetzung nach Ansprüchen 3 - 4 beim Ölbohren oder Fracking.

17. Verwendung der Variante nach Ansprüchen 1 - 2 oder der Enzymzusammensetzung nach Ansprüchen 3 - 4 beim Verarbeiten von Kaffeeextrakt, Fruchtsaft, Ananassaft oder Sojamilch.

## Revendications

1. Variant de mannanase présentant une activité mannanase et un acide aminé non glycosylé en position 283, dans lequel le variant est choisi dans le groupe constitué par
1) un polypeptide présentant au moins 85 % d'identité de séquence avec les résidus 27-331 de SEQ ID NO : 2 ;
2) un polypeptide codé par un polynucléotide qui s'hybride dans des conditions de stringence élevée avec
a) les nucléotides 79-993 de SEQ ID NO : 1 (*man7*) ou
b) le complément intégral de a), et dans lequel les conditions de stringence élevée correspondent à l'hybridation lors de la préhybridation et de l'hybridation à 65 °C dans 5x SSC, 0,1% N-lauroylsarcosine, 0,02% SDS, 1% réactif de blocage (Roche 11 096 176 001), en suivant les procédures standard de transfert de Southern pendant 12 à 24 heures, et en lavant deux à trois fois pendant 15 minutes à l'aide de 0,1 X SSC, 0,1% SDS à 65 °C ; et
3) un variant codé par un polynucléotide présentant au moins 95 % d'identité de séquence avec la SEQ ID NO : 1 ou sa séquence d'ADN génomique ; et
dans lequel la numérotation des acides aminés correspond à la numérotation des acides aminés de la séquence d'acides aminés pleine longueur SEQ ID NO : 2 (Man7) contenant une séquence signal, et dans lequel la position N283, T285 ou S285 est substituée par un résidu qui empêche la N-glycosylation du résidu 283 lorsqu'il est exprimé dans une cellule hôte capable de N-glycosylation.

2. Variant selon l'une quelconque de la revendication 1, dans lequel la position T285 ou S285 est substituée à un résidu autre que T ou S.

3. Composition enzymatique comprenant le variant de mannanase selon l'une quelconque des revendications 1 à 2 et
a. au moins un conservateur choisi par exemple dans le groupe constitué par l'acide organique, l'acide citrique, l'acide ascorbique, l'acide benzoïque et leurs sels et dérivés, le benzoate de sodium, l'hydroxybenzoate de benzoate et ses dérivés, l'acide sorbique, le sorbate de sodium, le sorbate, les sels, tels que le chlorure de sodium ou le chlorure de potassium, 1,2-Benzisothiazolin-3-one (BIT), ou une combinaison de ceux-ci ;
b. éventuellement au moins un stabilisant choisi parmi le polyol, le propylène glycol, le polyéthylène glycol, l'hexylène glycol, le glycérol, un sucre, l'alcool de sucre, le polysaccharide, l'acide lactique, l'acide borique, le dérivé d'acide borique, l'ester borate aromatique, l'acide 4-formylphényl boronique, le dérivé d'acide phényl boronique, un peptide, un tensioactif ou une combinaison de ceux-ci ;
c. éventuellement au moins une enzyme choisie parmi les protéases, les amylases, les cellulases, les lipases, les xylanases, les mannanases, les cutinases, les estérases, les phytases, les DNAses, les pectinases, les enzymes pectinolytiques, les lyases de pectate, les carbohydrases, les arabinases, les galactanases, les xanthanases, les xyloglucanases, les laccases, les peroxydases et oxydases avec ou sans médiateur, ou une combinaison de ceux-ci ; et
d. éventuellement au moins un diluant choisi parmi la maltodextrine, la farine, le chlorure de sodium, le sulfate, le sulfate de sodium ou une combinaison de ceux-ci.

4. Composition enzymatique selon la revendication 3 sous la forme d'une composition liquide ou d'une composition solide telle qu'une solution, une dispersion, une pâte, une poudre, une granule, un granulé, un granulé enrobé, un comprimé, un pain, un cristal, une bouillie cristalline, un gel ou une pastille.

5. Composition détergente comprenant le variant de mannanase selon l'une quelconque des revendications 1 à 2 ou la composition enzymatique des revendications 3 à 4.

6. Composition détergente selon la revendication 5 sous forme de détergent liquide ou de détergent solide de préférence sous forme de barre, de comprimé homogène, de comprimé à deux ou plusieurs couches, de pochette à un ou plusieurs compartiments, de poudre régulière ou compacte, de granule, de granulé, de pâte, de gel ou de liquide régulier, compact ou concentré.

7. Composition détergente selon la revendication 5 ou 6, comprenant en outre une ou plusieurs enzymes supplémentaires choisies dans le groupe constitué par une protéase, une lipase, une cutinase, une amylase, une carbohydrase, une cellulase, une pectinase, une lyase de pectate, une enzyme pectinolytique, une estérase, une mannanase, une arabinase, une galactanase, une xylanase, une oxydase, une xanthanase, une xyloglucanase, une laccase, une DNAse et/ou une peroxydase, de préférence choisies dans le groupe constitué par les protéases, les amylases, les cellulases et les lipases.

8. Cellule hôte recombinante comprenant des éléments génétiques qui permettent de produire au moins un polypeptide recombinant comprenant le variant de mannanase des revendications 1 à 2, et dans laquelle les éléments génétiques comprennent au moins une séquence de contrôle qui contrôle la production du polypeptide recombinant dans la cellule hôte recombinante.

9. Cellule hôte recombinante selon la revendication 8, dans laquelle la cellule hôte est choisie dans le groupe constitué de :
cellules fongiques, cellules fongiques filamenteuses de la division *Ascomycota,* de la sous-division *Pezizomycotina ;*
de préférence parmi le groupe composé de membres de la classe *Sordariomycetes,* de la sous-classe *Hypocreomycetidae,* des ordres *Hypocreales* et *Microascales* et *Aspergillus, Chrysosporium, Myceliophthora* et *Humicola ;*
plus préférablement dans le groupe composé des familles *Hypocreacea, Nectriaceae, Clavicipitaceae, Microascaceae* et Genera *Trichoderma* (anamorphe de *Hypocrea), Fusarium, Gibberella, Nectria, Stachybotrys, Claviceps, Metarhizium, Villosiclava, Ophiocordyceps, Cephalosporium* et *Scedosporium ;*
plus préférablement dans le groupe composé de *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. Pseudograminearum, F. venenatum, Gibberella fujikuroi, G. moniliformis,* G. zeaea, *Nectria (Haematonectria) haematococca, Stachybotrys chartarum, S. chlorohalonata, Claviceps purpurea, Metarhizium acridum, M. anisopliae, Villosicliums virens, Ophiocordyceps sinensis, Acremonium (Cephalosporium) chrysogenum,* et *Scedosporium apiospermum et Aspergillus niger, Aspergillus awamori, Aspergillus oryzae, Chrysosporium lucknowense, Myceliophthora thermophila, Humicola insolens* et *Humicola grisea,* cellules bactériennes, de préférence des bactéries bacilles à Gram positif tels que *B. subtilisis, B. licheniformis, B. megaterium, B. amyloliquefaciens, B. pumilus,* des bactéries à Gram négatif telles que *Escherichia coli,* des actinomycètes telles que *Streptomyces* sp. et des levures, telles que *Saccharomyces cerevisiae, Pichia pastoris, Yarrowia lipolytica,* de préférence *Trichoderma reesei.*

10. Cellule hôte recombinante selon l'une quelconque des revendications 8 à 9, dans laquelle le polypeptide recombinant est une protéine de fusion, qui comprend en outre au moins l'un des éléments suivants :
une séquence d'acides aminés fournissant une séquence signal sécrétoire ;
une séquence d'acides aminés qui facilite la purification, telle qu'une étiquette d'affinité ou une étiquette poly-histidine ;
une séquence d'acides aminés qui améliore la production, telle qu'une séquence d'acides aminés qui est un support, telle que des CBM ;
une séquence d'acides aminés présentant une activité enzymatique ; et
une séquence d'acides aminés fournissant à la protéine de fusion une affinité de liaison, telle qu'une fraction de liaison aux glucides.

11. Procédé de production d'un polypeptide recombinant présentant une activité mannanase comprenant :
a. la culture d'une cellule hôte recombinante selon l'une quelconque des revendications 8 à 10,
les éléments génétiques comprenant au moins une séquence de contrôle qui contrôle la production du polypeptide recombinant dans la cellule hôte recombinante
les éléments génétiques comprenant éventuellement au moins une séquence codant pour une séquence signal destinée à transporter le polypeptide recombinant hors de la cellule hôte ; et
la culture étant effectuée dans des conditions permettant la production du polypeptide recombinant ; et
a. la récupération du polypeptide recombinant.

12. Procédé de dégradation ou de modification d'une matière contenant du mannane, comprenant le traitement de ladite matière contenant du mannane avec une quantité efficace de la composition enzymatique de l'une quelconque des revendications 3 à 4 ou du variant de l'une quelconque des revendications 1 à 2.

13. Procédé selon la revendication 12, dans lequel la matière contenant du mannane est une matière, du textile, des eaux usées, des eaux d'égouts, de l'huile ou une combinaison de ceux-ci à base de plantes.

14. Aliment pour animaux comprenant la composition enzymatique de l'une quelconque des revendications 3 à 4 ou le variant de mannanase de l'une quelconque des revendications 1 à 2, et au moins une source de protéines d'origine végétale ou un produit ou sous-produit contenant du mannane, et
a. éventuellement, au moins une enzyme choisie parmi la protéase, l'amylase, la phytase, la xylanase, l'endoglucanase, la bêta-glucanase ou une combinaison de celles-ci ; et
b. éventuellement, au moins un diluant choisi parmi la maltodextrine, la farine, le sel, le chlorure de sodium, le sulfate, le sulfate de sodium ou une combinaison de ceux-ci.

15. Aliment complémentaire comprenant la composition enzymatique de l'une quelconque des revendications 3 à 4 ou le variant de mannanase de l'une quelconque des revendications 1 à 2 ; et
a. éventuellement, au moins une enzyme choisie parmi la protéase, l'amylase, la phytase, la xylanase, l'endoglucanase, la bêta-glucanase ou une combinaison de celles-ci ; et
b. éventuellement, au moins un diluant choisi parmi la maltodextrine, la farine, le sel, le chlorure de sodium, le sulfate, le sulfate de sodium ou une combinaison de ceux-ci.

16. Utilisation du variant des revendications 1 à 2 ou de la composition enzymatique des revendications 3 à 4 dans le forage pétrolier ou l'hydro-fracturation.

17. Utilisation du variant des revendications 1 à 2 ou de la composition enzymatique des revendications 3 à 4 dans le traitement d'extrait de café, de jus de fruits, de jus d'ananas ou de lait de soja.


### SEQUENCE LISTING

<110> AB Enzymes Oy
<120> Mannanase variants, G
<130> 31603EP-1f_ALLMAN-G
<160> 26
<170> PatentIn version 3.5
<210> 1
   <211> 1395
   <212> DNA
   <213> Bacillus hemicellulosilyticus
<400> 1
   cagacccact cgggcttcta catcgagggc tcgacgctct acgacgctaa cggcgagcct      60
   tttgtcatgc gcggcatcaa ccacggccac gcctggtaca agcacgactc caacgtcgct     120
   atccctgcta tcgctaacca gggcgctaac accatccgca tcgtcctcag cgacggtggc     180
   cagtgggcca aggacgacat caacacgctg aaccaggtcc tcgacctggc cgaggagcac     240
   gagatgatcg ctgtcgtcga ggtccacgac gctaccggct ccaacagcat ggccgacctc     300
   aaccgcgccg tcgactactg gatcgagatg aaggacgccc tgatcggcaa ggaagaccgc     360
   gtcatcatca acatcgctaa cgagtggtac ggcgcttggg acggccaggg ctgggccaac     420
   ggctacaagg aagtcatccc tcgcctgcgc aacgctggct tcacccacac cctcatggtc     480
   gacgctgccg gctggggcca gtaccctcag agcatccacg actacggcca agaggtcttc     540
   aacgccgacc ctctggccaa caccatgttc tccatccaca tgtacgagta cgctggcggc     600
   aacgcctcca tggtccagag caacatcgac ggcgtcgtcg accagggcct cgctctggtc     660
   atcggcgagt tcggccacat gcacacggac ggcgacgtcg acgaggctac catcctgagc     720
   tactcgcagc agcgcggcgt cggctggctg gcctggtcgt ggaagggcaa cggcacccag     780
   tgggagtacc tcgacctgag ctacgactgg cagggcacca acctcacgtc gtggggcaac     840
   acgatcgtcc acggccctaa cggcctcctg gagacgtcca tcccttccag catctttcac     900
   accgctccta acaacggcga ccctcctccc cacaacggca acgagacgat cctgtacgac     960
   ttcgagcacg gcacgcaggg ctggtcgggc tcgtccctgc tgggcggccc ttggaccacc    1020
   aacgagtggt cgaccaacgg caaccactcc ctcaaggccg acatcttcct gtccgccaac    1080
   agcaagcacg agctcgccaa ggtcgagaac cgcaacctca gcggctactc gacgctgcag    1140
   gctaccgtcc gccacgctca ctggggcaac gtcggcaacc tgacggctcg catgtacgtc    1200
   aagacgggca gcaactactc gtggttcaac ggcgacccca tccctgtcaa ctcggctaac    1260
   ggcaccaccg tcaccctccc tctgagctcg atccccaacc tcaacgacgt caaggagatc    1320
   ggcgtcgagt tcatcggcgc tagcaacagc aacggccaga ccgccatcta cctggaccac    1380
   gtcacgatcc agtaa                                                    1395
<210> 2
   <211> 490
   <212> PRT
   <213> Bacillus hemicellulosilyticus
<400> 2
   Met Arg Asn Phe Gly Lys Leu Ile Val Ser Ser Cys Leu Leu Phe Ser
   1               5                   10                  15
   Phe Phe Leu Phe Ala Ser Asp Gly His Ser Gln Thr His Ser Gly Phe
               20                  25                  30
   Tyr Ile Glu Gly Ser Thr Leu Tyr Asp Ala Asn Gly Glu Pro Phe Val
           35                  40                  45
   Met Arg Gly Ile Asn His Gly His Ala Trp Tyr Lys His Asp Ser Asn
       50                  55                  60
   Val Ala Ile Pro Ala Ile Ala Asn Gln Gly Ala Asn Thr Ile Arg Ile
   65                  70                  75                  80
   Val Leu Ser Asp Gly Gly Gln Trp Ala Lys Asp Asp Ile Asn Thr Leu
                   85                  90                  95
   Asn Gln Val Leu Asp Leu Ala Glu Glu His Glu Met Ile Ala Val Val
               100                 105                 110
   Glu Val His Asp Ala Thr Gly Ser Asn Ser Met Ala Asp Leu Asn Arg
           115                 120                 125
   Ala Val Asp Tyr Trp Ile Glu Met Lys Asp Ala Leu Ile Gly Lys Glu
       130                 135                 140
   Asp Arg Val Ile Ile Asn Ile Ala Asn Glu Trp Tyr Gly Ala Trp Asp
   145                 150                 155                 160
   Gly Gln Gly Trp Ala Asn Gly Tyr Lys Glu Val Ile Pro Arg Leu Arg
                   165                 170                 175
   Asn Ala Gly Phe Thr His Thr Leu Met Val Asp Ala Ala Gly Trp Gly
               180                 185                 190
   Gln Tyr Pro Gln Ser Ile His Asp Tyr Gly Gln Glu Val Phe Asn Ala
           195                 200                 205
   Asp Pro Leu Ala Asn Thr Met Phe Ser Ile His Met Tyr Glu Tyr Ala
       210                 215                 220
   Gly Gly Asn Ala Ser Met Val Gln Ser Asn Ile Asp Gly Val Val Asp
   225                 230                 235                 240
   Gln Gly Leu Ala Leu Val Ile Gly Glu Phe Gly His Met His Thr Asp
                   245                 250                 255
   Gly Asp Val Asp Glu Ala Thr Ile Leu Ser Tyr Ser Gln Gln Arg Gly
               260                 265                 270
   Val Gly Trp Leu Ala Trp Ser Trp Lys Gly Asn Gly Thr Gln Trp Glu
           275                 280                 285
   Tyr Leu Asp Leu Ser Tyr Asp Trp Gln Gly Thr Asn Leu Thr Ser Trp
       290                 295                 300
   Gly Asn Thr Ile Val His Gly Pro Asn Gly Leu Leu Glu Thr Ser Ile
   305                 310                 315                 320
   Pro Ser Ser Ile Phe His Thr Ala Pro Asn Asn Gly Asp Pro Pro Pro
                   325                 330                 335
   His Asn Gly Asn Glu Thr Ile Leu Tyr Asp Phe Glu His Gly Thr Gln
               340                 345                 350
   Gly Trp Ser Gly Ser Ser Leu Leu Gly Gly Pro Trp Thr Thr Asn Glu
           355                 360                 365
   Trp Ser Thr Asn Gly Asn His Ser Leu Lys Ala Asp Ile Phe Leu Ser
       370                 375                 380
   Ala Asn Ser Lys His Glu Leu Ala Lys Val Glu Asn Arg Asn Leu Ser
   385                 390                 395                 400
   Gly Tyr Ser Thr Leu Gln Ala Thr Val Arg His Ala His Trp Gly Asn
                   405                 410                 415
   Val Gly Asn Leu Thr Ala Arg Met Tyr Val Lys Thr Gly Ser Asn Tyr
               420                 425                 430
   Ser Trp Phe Asn Gly Asp Pro Ile Pro Val Asn Ser Ala Asn Gly Thr
           435                 440                 445
   Thr Val Thr Leu Pro Leu Ser Ser Ile Pro Asn Leu Asn Asp Val Lys
       450                 455                 460
   Glu Ile Gly Val Glu Phe Ile Gly Ala Ser Asn Ser Asn Gly Gln Thr
   465 470 475 480
   Ala Ile Tyr Leu Asp His Val Thr Ile Gln
   485 490
<210> 3
   <211> 464
   <212> PRT
   <213> Artificial Sequence
<220>
   <223> variant
<400> 3
   Gln Thr His Ser Gly Phe Tyr Ile Glu Gly Ser Thr Leu Tyr Asp Ala
   1 5 10 15
   Asn Gly Glu Pro Phe Val Met Arg Gly Ile Asn His Gly His Ala Trp
   20 25 30
   Tyr Lys His Asp Ser Asn Val Ala Ile Pro Ala Ile Ala Asn Gln Gly
   35 40 45
   Ala Asn Thr Ile Arg Ile Val Leu Ser Asp Gly Gly Gln Trp Ala Lys
   50 55 60
   Asp Asp Ile Asn Thr Leu Asn Gln Val Leu Asp Leu Ala Glu Glu His
   65 70 75 80
   Glu Met Ile Ala Val Val Glu Val His Asp Ala Thr Gly Ser Asn Ser
   85 90 95
   Met Ala Asp Leu Asn Arg Ala Val Asp Tyr Trp Ile Glu Met Lys Asp
   100 105 110
   Ala Leu Ile Gly Lys Glu Asp Arg Val Ile Ile Asn Ile Ala Asn Glu
   115 120 125
   Trp Tyr Gly Ala Trp Asp Gly Gln Gly Trp Ala Asn Gly Tyr Lys Glu
   130 135 140
   Val Ile Pro Arg Leu Arg Asn Ala Gly Phe Thr His Thr Leu Met Val
   145 150 155 160
   Asp Ala Ala Gly Trp Gly Gln Tyr Pro Gln Ser Ile His Asp Tyr Gly
   165 170 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ser Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Thr Gln Trp Glu Tyr Leu Asp Leu Ser Tyr Asp Trp Gln Gly
               260                 265                 270
   Thr Asn Leu Thr Ser Trp Gly Asn Thr Ile Val His Gly Pro Asn Gly
           275                 280                 285
   Leu Leu Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
   435 440 445
   Asn Ser Asn Gly Gln Thr Ala Ile Tyr Leu Asp His Val Thr Ile Gln
   450 455 460
<210> 4
   <211> 305
   <212> PRT
   <213> Artificial Sequence
<220>
   <223> variant
<400> 4
   Gln Thr His Ser Gly Phe Tyr Ile Glu Gly Ser Thr Leu Tyr Asp Ala
   1 5 10 15
   Asn Gly Glu Pro Phe Val Met Arg Gly Ile Asn His Gly His Ala Trp
   20 25 30
   Tyr Lys His Asp Ser Asn Val Ala Ile Pro Ala Ile Ala Asn Gln Gly
   35 40 45
   Ala Asn Thr Ile Arg Ile Val Leu Ser Asp Gly Gly Gln Trp Ala Lys
   50 55 60
   Asp Asp Ile Asn Thr Leu Asn Gln Val Leu Asp Leu Ala Glu Glu His
   65 70 75 80
   Glu Met Ile Ala Val Val Glu Val His Asp Ala Thr Gly Ser Asn Ser
   85 90 95
   Met Ala Asp Leu Asn Arg Ala Val Asp Tyr Trp Ile Glu Met Lys Asp
   100 105 110
   Ala Leu Ile Gly Lys Glu Asp Arg Val Ile Ile Asn Ile Ala Asn Glu
   115 120 125
   Trp Tyr Gly Ala Trp Asp Gly Gln Gly Trp Ala Asn Gly Tyr Lys Glu
   130 135 140
   Val Ile Pro Arg Leu Arg Asn Ala Gly Phe Thr His Thr Leu Met Val
   145 150 155 160
   Asp Ala Ala Gly Trp Gly Gln Tyr Pro Gln Ser Ile His Asp Tyr Gly
   165 170 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ser Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Thr Gln Trp Glu Tyr Leu Asp Leu Ser Tyr Asp Trp Gln Gly
               260                 265                 270
   Thr Asn Leu Thr Ser Trp Gly Asn Thr Ile Val His Gly Pro Asn Gly
           275                 280                 285
   Leu Leu Glu Thr Ser Ile Pro Ser Ser Ile Phe His Thr Ala Pro Asn
       290                 295                 300
   Asn
   305
<210> 5
   <211> 1395
   <212> DNA
   <213> Artificial Sequence
<220>
   <223> variant
<400> 5
   cagacccact ccggcttcta catcgagggc agcacgctct acgacgctaa cggcgagcct      60
   tttgtcatgc gcggcatcaa ccacggccac gcctggtaca agcacgactc caacgtcgcc     120
   atccctgcta tcgccaacca gggcgctaac accatccgca tcgtcctcag cgacggtggc     180
   cagtgggcca aggacgacat caacacgctg aaccaggtcc tcgacctggc cgaggagcac     240
   gagatgatcg ctgtcgtcga ggtccacgac gctaccggca gcaactcgat cgccgacctc     300
   aaccgcgccg tcgactactg gatcgagatg aaggacgccc tgatcggcaa ggaagaccgc     360
   gtcatcatca acatcgccaa cgagtggtac ggcgcttggg acggccaggg ctgggccaac     420
   ggctacaagg aagtcatccc ccgcctgcgc aacgctggct tcacccacac cctcatggtc     480
   gacgctgctg gctggggcca gtacccccag agcatccacg actacggcca agaggtcttc     540
   aacgccgacc ctctggccaa caccatgttc agcatccaca tgtacgagta cgctggcggc     600
   aacgctgcta tggtccagtc gaacatcgac ggcgtcgtcg accagggcct cgctctggtc     660
   atcggcgagt tcggccacat gcacacggac ggcgacgtcg acgaggctac catcctctcg     720
   tactcgcagc agcgccaggt cggctggctc gcttggagct ggaagggcaa cggcgctcag     780
   tgggagtacc tcgacctgtc gtacgactgg cagggcacca acctcacgtc ctggggcaac     840
   accatcgtcc acggccctaa cggcctcctg gagacgtcga tccctagctc gatctttcac     900
   accgctccta acaacggcga ccctcctccc cacaacggca acgagacgat cctgtacgac     960
   ttcgagcacg gcacgcaggg ctggtcgggc tccagcctgc tgggcggccc ttggaccacc    1020
   aacgagtgga gcacgaacgg caaccactcg ctcaaggccg acatcttcct gagcgccaac    1080
   tcgaagcacg agctcgccaa ggtcgagaac cgcaacctct cgggctactc gacgctgcag    1140
   gctaccgtcc gccacgctca ctggggcaac gtcggcaacc tgacggctcg catgtacgtc    1200
   aagacgggct cgaactactc ctggttcaac ggcgacccca tccctgtcaa ctcggctaac    1260
   ggcaccaccg tcaccctccc tctgagctcc atccccaacc tcaacgacgt caaggagatc    1320
   ggcgtcgagt tcatcggcgc tagcaactcg aacggccaga ccgccatcta cctggaccac    1380
   gtcacgatcc agtag                                                     1395
<210> 6
   <211> 464
   <212> PRT
   <213> Artificial Sequence
<220>
   <223> variant
<400> 6
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Ile Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ala Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ala Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gln Val Gly Trp Leu Ala Trp Ser Trp Lys Gly
                   245                 250                 255
   Asn Gly Ala Gln Trp Glu Tyr Leu Asp Leu Ser Tyr Asp Trp Gln Gly
               260                 265                 270
   Thr Asn Leu Thr Ser Trp Gly Asn Thr Ile Val His Gly Pro Asn Gly
           275                 280                 285
   Leu Leu Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
   340 345 350
   Ala Asp Ile Phe Leu Ser Ala Asn Ser Lys His Glu Leu Ala Lys Val
   355 360 365
   Glu Asn Arg Asn Leu Ser Gly Tyr Ser Thr Leu Gln Ala Thr Val Arg
   370 375 380
   His Ala His Trp Gly Asn Val Gly Asn Leu Thr Ala Arg Met Tyr Val
   385 390 395 400
   Lys Thr Gly Ser Asn Tyr Ser Trp Phe Asn Gly Asp Pro Ile Pro Val
   405 410 415
   Asn Ser Ala Asn Gly Thr Thr Val Thr Leu Pro Leu Ser Ser Ile Pro
   420 425 430
   Asn Leu Asn Asp Val Lys Glu Ile Gly Val Glu Phe Ile Gly Ala Ser
   435 440 445
   Asn Ser Asn Gly Gln Thr Ala Ile Tyr Leu Asp His Val Thr Ile Gln
   450 455 460
<210> 7
   <211> 1395
   <212> DNA
   <213> Artificial Sequence
<220>
   <223> variant
<400> 7
   cagacccact ccggcttcta catcgagggc agcacgctct acgacgctaa cggcgagcct        60
   tttgtcatgc gcggcatcaa ccacggccac gcctggtaca agcacgactc caacgtcgcc       120
   atccctgcta tcgccaacca gggcgctaac accatccgca tcgtcctcag cgacggtggc       180
   cagtgggcca aggacgacat caacacgctg aaccaggtcc tcgacctggc cgaggagcac       240
   gagatgatcg ctgtcgtcga ggtccacgac gctaccggca gcaactcgat ggccgacctc       300
   aaccgcgccg tcgactactg gatcgagatg aaggacgccc tgatcggcaa ggaagaccgc       360
   gtcatcatca acatcgccaa cgagtggtac ggcagctggg acggccaggg ctgggccaac       420
   ggctacaagg aagtcatccc ccgcctgcgc aacgctggct tcacccacac cctcatggtc       480
   gacgctgctg gctggggcca gtacccccag tcgatccacg actacggcca agaggtcttc       540
   aacgccgacc ctctggccaa caccatgttc agcatccaca tgtacgagta cgctggcggc       600
   aacgctgcta tggtccagtc gaacatcgac ggcgtcgtcg accagggcct cgctctggtc       660
   atcggcgagt tcggccacat gcacacggac ggcgacgtcg acgaggctac catcctgtcc       720
   tactcgcagc agcgcggcgt cggctggctc gcttggtcgt ggaagggcaa cggcgctcag       780
   tgggagtacc tcgacctgtc gtacgactgg cagggcacca acctcacctc gtggggcaac       840
   cgcatcgtcc acggccctaa cggcctcctg gagacgtcga tccctagctc gatctttcac       900
   accgctccta acaacggcga ccctcctccc cacaacggca acgagacgat cctgtacgac       960
   ttcgagcacg gcacgcaggg ctggtcgggc tccagcctgc tgggcggccc ttggaccacc      1020
   aacgagtgga gcaccaacgg caaccactcg ctcaaggccg acatcttcct gagcgccaac      1080
   tcgaagcacg agctcgccaa ggtcgagaac cgcaacctct cgggctactc gacgctgcag      1140
   gctaccgtcc gccacgctca ctggggcaac gtcggcaacc tgacggctcg catgtacgtc      1200
   aagacgggct cgaactactc ctggttcaac ggcgacccca tccctgtcaa ctcggctaac      1260
   ggcaccaccg tcaccctccc tctgagctcc atccccaacc tcaacgacgt caaggagatc      1320
   ggcgtcgagt tcatcggcgc tagcaactcg aacggccaga ccgccatcta cctggaccac      1380
   gtcacgatcc agtag                                                       1395
<210> 8
   <211> 464
   <212> PRT
   <213> Artificial Sequence
<220>
   <223> variant
<400> 8
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ser Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ala Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Ala Gln Trp Glu Tyr Leu Asp Leu Ser Tyr Asp Trp Gln Gly
               260                 265                 270
   Thr Asn Leu Thr Ser Trp Gly Asn Arg Ile Val His Gly Pro Asn Gly
           275                 280                 285
   Leu Leu Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460
<210> 9
   <211> 1395
   <212> DNA
   <213> Artificial Sequence
<220>
   <223> variant
<400> 9
   cagacccact ccggcttcta catcgagggc agcacgctct acgacgctaa cggcgagcct      60
   tttgtcatgc gcggcatcaa ccacggccac gcctggtaca agcacgactc caacgtcgcc     120
   atccctgcta tcgccaacca gggcgctaac accatccgca tcgtcctcag cgacggtggc     180
   cagtgggcca aggacgacat caacacgctg aaccaggtcc tcgacctggc cgaggagcac     240
   gagatgatcg ctgtcgtcga ggtccacgac gctaccggca gcaactcgat ggccgacctc     300
   aaccgcgccg tcgactactg gatcgagatg aaggacgccc tgatcggcaa ggaagaccgc     360
   gtcatcatca acatcgccaa cgagtggtac ggcgcttggg acggccaggg ctgggccaac     420
   ggctacaagg aagtcatccc ccgcctgcgc aacgctggct tcacccacac cctcatggtc     480
   gacgctgctg gctggggcca gtacccccag agcatccacg actacggcca agaggtcttc     540
   aacgccgacc ctctcgccaa caccatgttc agcatccaca tgtacgagta cgctggcggc     600
   aacgctgcta tggtccagtc gaacatcgac ggcgtcgtcg accagggcct cgctctggtc     660
   atcggcgagt tcggccacat gcacacggac ggcgacgtcg acgaggctac catcctgtcc     720
   tactcgcagc agcgcggcgt cggctggctc gcttggagct ggaagggcaa cggcgctcag     780
   tgggagtacc tcgacctgtc gtacgactgg cagggcacca acctcacgtc ctggggcaac     840
   accatcgtcc acggccctaa cggcctgaag gagacgtcga tccctagctc gatctttcac      900
   accgctccta acaacggcga ccctcctccc cacaacggca acgagacgat cctgtacgac      960
   ttcgagcacg gcacgcaggg ctggtcgggc tccagcctgc tgggcggccc ttggaccacc     1020
   aacgagtgga gcacgaacgg caaccactcg ctcaaggccg acatcttcct gagcgccaac     1080
   tcgaagcacg agctcgccaa ggtcgagaac cgcaacctct cgggctactc gacgctgcag     1140
   gctaccgtcc gccacgctca ctggggcaac gtcggcaacc tgacggctcg catgtacgtc     1200
   aagacgggct cgaactactc ctggttcaac ggcgacccca tccctgtcaa ctcggctaac     1260
   ggcaccaccg tcaccctccc tctgagctcc atccccaacc tcaacgacgt caaggagatc     1320
   ggcgtcgagt tcatcggcgc tagcaactcg aacggccaga ccgccatcta cctggaccac     1380
   gtcacgatcc agtag                                                      1395
<210> 10
   <211> 464
   <212> PRT
   <213> Artificial Sequence
<220>
   <223> variant
<400> 10
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ala Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ala Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Ala Gln Trp Glu Tyr Leu Asp Leu Ser Tyr Asp Trp Gln Gly
               260                 265                 270
   Thr Asn Leu Thr Ser Trp Gly Asn Thr Ile Val His Gly Pro Asn Gly
           275                 280                 285
   Leu Lys Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460
<210> 11
   <211> 1395
   <212> DNA
   <213> Artificial Sequence
<220>
   <223> variant
<400> 11
   cagacccact ccggcttcta catcgagggc agcacgctct acgacgctaa cggcgagcct      60
   tttgtcatgc gcggcatcaa ccacggccac gcctggtaca agcacgactc caacgtcgcc     120
   atccctgcta tcgccaacca gggcgctaac accatccgca tcgtcctcag cgacggtggc     180
   cagtgggcca aggacgacat caacacgctg aaccaggtcc tcgacctggc cgaggagcac     240
   gagatgatcg ctgtcgtcga ggtccacgac gctaccggca gcaactcgat cgccgacctc     300
   aaccgcgccg tcgactactg gatcgagatg aaggacgccc tgatcggcaa ggaagaccgc     360
   gtcatcatca acatcgccaa cgagtggtac ggctcttggg acggccaggg ctgggccaac     420
   ggctacaagg aagtcatccc ccgcctgcgc aacgctggct tcacccacac cctcatggtc     480
   gacgctgctg gctggggcca gtacccccag agcatccacg actacggcca agaggtcttc     540
   aacgccgacc ctctggccaa caccatgttc agcatccaca tgtacgagta cgctggcggc     600
   aacgctgcta tggtccagtc gaacatcgac ggcgtcgtcg accagggcct cgctctggtc     660
   atcggcgagt tcggccacat gcacacggac ggcgacgtcg acgaggctac catcctctcg     720
   tactcgcagc agcgccaggt cggctggctc gcttggagct ggaagggcaa cggcgctcag     780
   tgggagtacc tcgacctgtc gtacgactgg cagggcacca acctcacgtc ctggggcaac     840
   agaatcgtcc acggccctaa cggcctcctg gagacgtcga tccctagctc gatctttcac     900
   accgctccta acaacggcga ccctcctccc cacaacggca acgagacgat cctgtacgac     960
   ttcgagcacg gcacgcaggg ctggtcgggc tccagcctgc tgggcggccc ttggaccacc    1020
   aacgagtgga gcacgaacgg caaccactcg ctcaaggccg acatcttcct gagcgccaac    1080
   tcgaagcacg agctcgccaa ggtcgagaac cgcaacctct cgggctactc gacgctgcag    1140
   gctaccgtcc gccacgctca ctggggcaac gtcggcaacc tgacggctcg catgtacgtc    1200
   aagacgggct cgaactactc ctggttcaac ggcgacccca tccctgtcaa ctcggctaac    1260
   ggcaccaccg tcaccctccc tctgagctcc atccccaacc tcaacgacgt caaggagatc    1320
   ggcgtcgagt tcatcggcgc tagcaactcg aacggccaga ccgccatcta cctggaccac    1380
   gtcacgatcc agtag                                                     1395
<210> 12
   <211> 464
   <212> PRT
   <213> Artificial Sequence
<220>
   <223> variant
<400> 12
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Ile Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ser Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145 150 155 160
   Asp Ala Ala Gly Trp Gly Gln Tyr Pro Gln Ser Ile His Asp Tyr Gly
   165 170 175
   Gln Glu Val Phe Asn Ala Asp Pro Leu Ala Asn Thr Met Phe Ser Ile
   180 185 190
   His Met Tyr Glu Tyr Ala Gly Gly Asn Ala Ala Met Val Gln Ser Asn
   195 200 205
   Ile Asp Gly Val Val Asp Gln Gly Leu Ala Leu Val Ile Gly Glu Phe
   210 215 220
   Gly His Met His Thr Asp Gly Asp Val Asp Glu Ala Thr Ile Leu Ser
   225 230 235 240
   Tyr Ser Gln Gln Arg Gln Val Gly Trp Leu Ala Trp Ser Trp Lys Gly
   245 250 255
   Asn Gly Ala Gln Trp Glu Tyr Leu Asp Leu Ser Tyr Asp Trp Gln Gly
   260 265 270
   Thr Asn Leu Thr Ser Trp Gly Asn Arg Ile Val His Gly Pro Asn Gly
   275 280 285
   Leu Leu Glu Thr Ser Ile Pro Ser Ser Ile Phe His Thr Ala Pro Asn
   290 295 300
   Asn Gly Asp Pro Pro Pro His Asn Gly Asn Glu Thr Ile Leu Tyr Asp
   305 310 315 320
   Phe Glu His Gly Thr Gln Gly Trp Ser Gly Ser Ser Leu Leu Gly Gly
   325 330 335
   Pro Trp Thr Thr Asn Glu Trp Ser Thr Asn Gly Asn His Ser Leu Lys
   340 345 350
   Ala Asp Ile Phe Leu Ser Ala Asn Ser Lys His Glu Leu Ala Lys Val
   355 360 365
   Glu Asn Arg Asn Leu Ser Gly Tyr Ser Thr Leu Gln Ala Thr Val Arg
   370 375 380
   His Ala His Trp Gly Asn Val Gly Asn Leu Thr Ala Arg Met Tyr Val
   385 390 395 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460
<210> 13
   <211> 1395
   <212> DNA
   <213> Artificial Sequence
<220>
   <223> variant
<400> 13
   cagacccact ccggcttcta catcgagggc agcacgctct acgacgctaa cggcgagcct      60
   tttgtcatgc gcggcatcaa ccacggccac gcctggtaca agcacgactc caacgtcgcc     120
   atccctgcta tcgccaacca gggcgctaac accatccgca tcgtcctcag cgacggtggc     180
   cagtgggcca aggacgacat caacacgctg aaccaggtcc tcgacctggc cgaggagcac     240
   gagatgatcg ctgtcgtcga ggtccacgac gctaccggca gcaactcgat cgccgacctc     300
   aaccgcgccg tcgactactg gatcgagatg aaggacgccc tgatcggcaa ggaagaccgc     360
   gtcatcatca acatcgccaa cgagtggtac ggcgcttggg acggccaggg ctgggccaac     420
   ggctacaagg aagtcatccc ccgcctgcgc aacgctggct tcacccacac cctcatggtc     480
   gacgctgctg gctggggcca gtacccccag agcatccacg actacggcca agaggtcttc     540
   aacgccgacc ctctggccaa caccatgttc agcatccaca tgtacgagta cgctggcggc     600
   aacgctgcta tggtccagtc gaacatcgac ggcgtcgtcg accagggcct cgctctggtc     660
   atcggcgagt tcggccacat gcacacggac ggcgacgtcg acgaggctac catcctctcg     720
   tactcgcagc agcgccaggt cggctggctc gcttggagct ggaagggcaa cggcgctcag     780
   tgggagtacc tcgacctgtc gtacgactgg cagggcacca acctcacgtc ctggggcaac     840
   accatcgtcc acggccctaa cggcctcaaa gagacgtcga tccctagctc gatctttcac     900
   accgctccta acaacggcga ccctcctccc cacaacggca acgagacgat cctgtacgac     960
   ttcgagcacg gcacgcaggg ctggtcgggc tccagcctgc tgggcggccc ttggaccacc    1020
   aacgagtgga gcacgaacgg caaccactcg ctcaaggccg acatcttcct gagcgccaac    1080
   tcgaagcacg agctcgccaa ggtcgagaac cgcaacctct cgggctactc gacgctgcag    1140
   gctaccgtcc gccacgctca ctggggcaac gtcggcaacc tgacggctcg catgtacgtc    1200
   aagacgggct cgaactactc ctggttcaac ggcgacccca tccctgtcaa ctcggctaac    1260
   ggcaccaccg tcaccctccc tctgagctcc atccccaacc tcaacgacgt caaggagatc    1320
   ggcgtcgagt tcatcggcgc tagcaactcg aacggccaga ccgccatcta cctggaccac    1380
   gtcacgatcc agtag                                                     1395
<210> 14
   <211> 464
   <212> PRT
   <213> Artificial Sequence
<220>
   <223> variant
<400> 14
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Ile Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ala Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ala Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gln Val Gly Trp Leu Ala Trp Ser Trp Lys Gly
                   245                 250                 255
   Asn Gly Ala Gln Trp Glu Tyr Leu Asp Leu Ser Tyr Asp Trp Gln Gly
               260                 265                 270
   Thr Asn Leu Thr Ser Trp Gly Asn Thr Ile Val His Gly Pro Asn Gly
           275                 280                 285
   Leu Lys Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
   420 425 430
   Asn Leu Asn Asp Val Lys Glu Ile Gly Val Glu Phe Ile Gly Ala Ser
   435 440 445
   Asn Ser Asn Gly Gln Thr Ala Ile Tyr Leu Asp His Val Thr Ile Gln
   450 455 460
<210> 15
   <211> 1395
   <212> DNA
   <213> Artificial Sequence
<220>
   <223> variant
<400> 15
   cagacccact ccggcttcta catcgagggc agcacgctct acgacgctaa cggcgagcct 60
   tttgtcatgc gcggcatcaa ccacggccac gcctggtaca agcacgactc caacgtcgcc 120
   atccctgcta tcgccaacca gggcgctaac accatccgca tcgtcctcag cgacggtggc 180
   cagtgggcca aggacgacat caacacgctg aaccaggtcc tcgacctggc cgaggagcac 240
   gagatgatcg ctgtcgtcga ggtccacgac gctaccggca gcaactcgat ggccgacctc 300
   aaccgcgccg tcgactactg gatcgagatg aaggacgccc tgatcggcaa ggaagaccgc 360
   gtcatcatca acatcgccaa cgagtggtac ggcagctggg acggccaggg ctgggccaac 420
   ggctacaagg aagtcatccc ccgcctgcgc aacgctggct tcacccacac cctcatggtc 480
   gacgctgctg gctggggcca gtacccccag tcgatccacg actacggcca agaggtcttc 540
   aacgccgacc ctctggccaa caccatgttc agcatccaca tgtacgagta cgctggcggc 600
   aacgctgcta tggtccagtc gaacatcgac ggcgtcgtcg accagggcct cgctctggtc 660
   atcggcgagt tcggccacat gcacacggac ggcgacgtcg acgaggctac catcctgtcc 720
   tactcgcagc agcgcggcgt cggctggctc gcttggtcgt ggaagggcaa cggcgctcag 780
   tgggagtacc tcgacctgtc gtacgactgg cagggcacca acctcacctc gtggggcaac 840
   cgcatcgtcc acggccctaa cggcctcaaa gagacgtcga tccctagctc gatctttcac 900
   accgctccta acaacggcga ccctcctccc cacaacggca acgagacgat cctgtacgac 960
   ttcgagcacg gcacgcaggg ctggtcgggc tccagcctgc tgggcggccc ttggaccacc 1020
   aacgagtgga gcaccaacgg caaccactcg ctcaaggccg acatcttcct gagcgccaac 1080
   tcgaagcacg agctcgccaa ggtcgagaac cgcaacctct cgggctactc gacgctgcag 1140
   gctaccgtcc gccacgctca ctggggcaac gtcggcaacc tgacggctcg catgtacgtc 1200
   aagacgggct cgaactactc ctggttcaac ggcgacccca tccctgtcaa ctcggctaac 1260
   ggcaccaccg tcaccctccc tctgagctcc atccccaacc tcaacgacgt caaggagatc 1320
   ggcgtcgagt tcatcggcgc tagcaactcg aacggccaga ccgccatcta cctggaccac 1380
   gtcacgatcc agtag 1395
<210> 16
   <211> 464
   <212> PRT
   <213> Artificial Sequence
<220>
   <223> variant
<400> 16
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ser Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ala Met Val Gln Ser Asn
   195 200 205
   Ile Asp Gly Val Val Asp Gln Gly Leu Ala Leu Val Ile Gly Glu Phe
   210 215 220
   Gly His Met His Thr Asp Gly Asp Val Asp Glu Ala Thr Ile Leu Ser
   225 230 235 240
   Tyr Ser Gln Gln Arg Gly Val Gly Trp Leu Ala Trp Ser Trp Lys Gly
   245 250 255
   Asn Gly Ala Gln Trp Glu Tyr Leu Asp Leu Ser Tyr Asp Trp Gln Gly
   260 265 270
   Thr Asn Leu Thr Ser Trp Gly Asn Arg Ile Val His Gly Pro Asn Gly
   275 280 285
   Leu Lys Glu Thr Ser Ile Pro Ser Ser Ile Phe His Thr Ala Pro Asn
   290 295 300
   Asn Gly Asp Pro Pro Pro His Asn Gly Asn Glu Thr Ile Leu Tyr Asp
   305 310 315 320
   Phe Glu His Gly Thr Gln Gly Trp Ser Gly Ser Ser Leu Leu Gly Gly
   325 330 335
   Pro Trp Thr Thr Asn Glu Trp Ser Thr Asn Gly Asn His Ser Leu Lys
   340 345 350
   Ala Asp Ile Phe Leu Ser Ala Asn Ser Lys His Glu Leu Ala Lys Val
   355 360 365
   Glu Asn Arg Asn Leu Ser Gly Tyr Ser Thr Leu Gln Ala Thr Val Arg
   370 375 380
   His Ala His Trp Gly Asn Val Gly Asn Leu Thr Ala Arg Met Tyr Val
   385 390 395 400
   Lys Thr Gly Ser Asn Tyr Ser Trp Phe Asn Gly Asp Pro Ile Pro Val
   405 410 415
   Asn Ser Ala Asn Gly Thr Thr Val Thr Leu Pro Leu Ser Ser Ile Pro
   420 425 430
   Asn Leu Asn Asp Val Lys Glu Ile Gly Val Glu Phe Ile Gly Ala Ser
   435 440 445
   Asn Ser Asn Gly Gln Thr Ala Ile Tyr Leu Asp His Val Thr Ile Gln
   450 455 460
<210> 17
   <211> 1395
   <212> DNA
   <213> Artificial Sequence
<220>
   <223> variant
<400> 17
   cagacccact ccggcttcta catcgagggc agcacgctct acgacgctaa cggcgagcct 60
   tttgtcatgc gcggcatcaa ccacggccac gcctggtaca agcacgactc caacgtcgcc 120
   atccctgcta tcgccaacca gggcgctaac accatccgca tcgtcctcag cgacggtggc 180
   cagtgggcca aggacgacat caacacgctg aaccaggtcc tcgacctggc cgaggagcac 240
   gagatgatcg ctgtcgtcga ggtccacgac gctaccggca gcaactcgat ggccgacctc 300
   aaccgcgccg tcgactactg gatcgagatg aaggacgccc tgatcggcaa ggaagaccgc 360
   gtcatcatca acatcgccaa cgagtggtac ggcgcttggg acggccaggg ctgggccaac 420
   ggctacaagg aagtcatccc ccgcctgcgc aacgctggct tgacccacac cctcatggtc 480
   gacgctgctg gctggggcca gtacccccag agcatccacg actacggcca agaggtcttc 540
   aacgccgacc ctctcgccaa caccatgttc agcatccaca tgtacgagta cgctggcggc 600
   aacgctgcta tggtccagtc gaacatcgac ggcgtcgtcg accagggcct cgctctggtc 660
   atcggcgagt tcggccacat gcacacggac ggcgacgtcg acgaggctac catcctgtcc 720
   tactcgcagc agcgcggcgt cggctggctc gcttggagct ggaagggcaa cggcgctcag 780
   tgggagtacc tcgacctgtc gtacgactgg cagggcacca acctcacgtc ctggggcaac 840
   accatcgtcc acggccctaa cggcctgaag gagacgtcga tccctagctc gatctttcac 900
   accgctccta acaacggcga ccctcctccc cacaacggca acgagacgat cctgtacgac 960
   ttcgagcacg gcacgcaggg ctggtcgggc tccagcctgc tgggcggccc ttggaccacc 1020
   aacgagtgga gcacgaacgg caaccactcg ctcaaggccg acatcttcct gagcgccaac 1080
   tcgaagcacg agctcgccaa ggtcgagaac cgcaacctct cgggctactc gacgctgcag 1140
   gctaccgtcc gccacgctca ctggggcaac gtcggcaacc tgacggctcg catgtacgtc 1200
   aagacgggct cgaactactc ctggttcaac ggcgacccca tccctgtcaa ctcggctaac 1260
   ggcaccaccg tcaccctccc tctgagctcc atccccaacc tcaacgacgt caaggagatc 1320
   ggcgtcgagt tcatcggcgc tagcaactcg aacggccaga ccgccatcta cctggaccac 1380
   gtcacgatcc agtag 1395
<210> 18
   <211> 464
   <212> PRT
   <213> Artificial Sequence
<220>
   <223> variant
<400> 18
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ala Trp Asp Gly Gln Gly Trp Ala Asn Gly Tyr Lys Glu
       130                 135                 140
   Val Ile Pro Arg Leu Arg Asn Ala Gly Leu Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ala Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Ala Gln Trp Glu Tyr Leu Asp Leu Ser Tyr Asp Trp Gln Gly
               260                 265                 270
   Thr Asn Leu Thr Ser Trp Gly Asn Thr Ile Val His Gly Pro Asn Gly
           275                 280                 285
   Leu Lys Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460
<210> 19
   <211> 1395
   <212> DNA
   <213> Artificial Sequence
<220>
   <223> variant
<400> 19
   cagacccact ccggcttcta catcgagggc agcacgctct acgacgctaa cggcgagcct      60
   tttgtcatgc gcggcatcaa ccacggccac gcctggtaca agcacgactc caacgtcgcc     120
   atccctgcta tcgccaacca gggcgctaac accatccgca tcgtcctcag cgacggtggc     180
   cagtgggcca aggacgacat caacacgctg aaccaggtcc tcgacctggc cgaggagcac     240
   gagatgatcg ctgtcgtcga ggtccacgac gctaccggca gcaactcgat ggccgacctc     300
   aaccgcgccg tcgactactg gatcgagatg aaggacgccc tgatcggcaa ggaagaccgc     360
   gtcatcatca acatcgccaa cgagtggtac ggcgcttggg acggccaggg ctgggccaac     420
   ggctacaagg aagtcatccc ccgcctgcgc aacgctggct tcacccacac cctcatggtc     480
   gacgctgctg gctggggcca gtacccccag agcatccacg actacggcca agaggtcttc     540
   aacgccgacc ctctcgccaa caccatgttc agcatccaca tgtacgagta cgctggcggc     600
   aacgctgcta tggtccagtc gaacatcgac ggcgtcgtcg accagggcct cgctctggtc     660
   atcggcgagt tcggccacat gcacacggac ggcgacgtcg acgaggctac catcctgtcc     720
   tactcgcagc agcgcggcgt cggctggctc gcttggagct ggaagggcaa cggcgctcag     780
   tgggagtacc tcgacctgtc gtacgactgg cagggcacca acctcacgtc ctggggcaac     840
   accatcgtcc acggccctaa cggcctgttg gagacgtcga tccctagctc gatctttcac     900
   accgctccta acaacggcga ccctcctccc cacaacggca acgagacgat cctgtacgac     960
   ttcgagcacg gcacgcaggg ctggtcgggc tccagcctgc tgggcggccc ttggaccacc    1020
   aacgagtgga gcacgaacgg caaccactcg ctcaaggccg acatcttcct gagcgccaac    1080
   tcgaagcacg agctcgccaa ggtcgagaac cgcaacctct cgggctactc gacgctgcag    1140
   gctaccgtcc gccacgctca ctggggcaac gtcggcaacc tgacggctcg catgtacgtc    1200
   aagacgggct cgaactactc ctggttcaac ggcgacccca tccctgtcaa ctcggctaac    1260
   ggcaccaccg tcaccctccc tctgagctcc atccccaacc tcaacgacgt caaggagatc    1320
   ggcgtcgagt tcatcggcgc tagcaactcg aacggccaga ccgccatcta cctggaccac    1380
   gtcacgatcc agtag                                                    1395
<210> 20
   <211> 464
   <212> PRT
   <213> Artificial Sequence
<220>
   <223> variant
<400> 20
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ala Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ala Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Ala Gln Trp Glu Tyr Leu Asp Leu Ser Tyr Asp Trp Gln Gly
               260                 265                 270
   Thr Asn Leu Thr Ser Trp Gly Asn Thr Ile Val His Gly Pro Asn Gly
           275                 280                 285
   Leu Leu Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460
<210> 21
   <211> 1395
   <212> DNA
   <213> Artificial Sequence
<220>
   <223> variant
<400> 21
   cagacccact cgggcttcta catcgagggc tccacgctct acgacgctaa cggcgagcct      60
   tttgtcatgc gcggcatcaa ccacggccac gcctggtaca agcacgacag caacgtcgcc     120
   atccctgcta tcgccaacca gggcgctaac accatccgca tcgtcctctc ggacggtggc     180
   cagtgggcca aggacgacat caacacgctg aaccaggtcc tcgacctggc cgaggagcac     240
   gagatgatcg ctgtcgtcga ggtccacgac gctaccggca gcaactcgat ggccgacctc     300
   aaccgcgccg tcgactactg gatcgagatg aaggacgccc tgatcggcaa ggaagaccgc     360
   gtcatcatca acatcgccaa cgagtggtac ggcgcttggg acggccaggg ctgggccaac     420
   ggctacaagg aagtcatccc ccgcctgcgc aacgctggct tcacccacac cctcatggtc     480
   gacgctgctg gctggggcca gtacccccag tcgatccacg actacggcca agaggtcttc     540
   aacgccgacc ctctggccaa caccatgttc tcgatccaca tgtacgagta cgctggcggc     600
   aacgctgcta tggtccagtc caacatcgac ggcgtcgtcg accagggcct cgctctggtc     660
   atcggcgagt tcggccacat gcacacggac ggcgacgtcg acgaggctac catcctgtcc     720
   tactcgcagc agcgcggcgt cggctggctc gcttggtcgt ggaagggcaa cggcgctcag     780
   tgggagtacc tcgacctgag ctacgactgg cagggcaccc agctcaccag ctggggcaac     840
   accatcgtcc acggccctaa cggcctcctg gagacgtcca tccctagctc gatctttcac     900
   accgctccta acaacggcga ccctcctccc cacaacggcc aggagacgat cctgtacgac     960
   ttcgagcacg gcacgcaggg ctggtcgggc tccagcctgc tgggcggccc ttggaccacc    1020
   aacgagtggt ccacgaacgg caaccacagc ctcaaggccg acatcttcct gagcgccaac    1080
   tcgaagcacg agctcgccaa ggtcgagaac cgcaacctgg ctggctactc gacgctgcag    1140
   gctaccgtcc gccacgctca ctggggcaac gtcggcgctc tgacggctcg catgtacgtc    1200
   aagacgggct ccaactacgc ctggttcaac ggcgacccca tccctgtcaa ctcggctcag    1260
   ggcaccaccg tcaccctccc tctgagctcc atccccaacc tcaacgacgt caaggagatc    1320
   ggcgtcgagt tcatcggcgc tagcaactcg aacggccaga ccgccatcta cctggaccac    1380
   gtcacgatcc agtag                                                     1395
<210> 22
   <211> 464
   <212> PRT
   <213> Artificial Sequence
<220>
   <223> variant
<400> 22
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ala Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ala Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Ala Gln Trp Glu Tyr Leu Asp Leu Ser Tyr Asp Trp Gln Gly
               260                 265                 270
   Thr Gln Leu Thr Ser Trp Gly Asn Thr Ile Val His Gly Pro Asn Gly
           275                 280                 285
   Leu Leu Glu Thr Ser Ile Pro Ser Ser Ile Phe His Thr Ala Pro Asn
       290                 295                 300
   Asn Gly Asp Pro Pro Pro His Asn Gly Gln Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ala Gly Tyr Ser Thr Leu Gln Ala Thr Val Arg
       370                 375                 380
   His Ala His Trp Gly Asn Val Gly Ala Leu Thr Ala Arg Met Tyr Val
   385                 390                 395                 400
   Lys Thr Gly Ser Asn Tyr Ala Trp Phe Asn Gly Asp Pro Ile Pro Val
                   405                 410                 415
   Asn Ser Ala Gln Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460
<210> 23
   <211> 1395
   <212> DNA
   <213> Artificial Sequence
<220>
   <223> variant
<400> 23
   cagacccact ccggcttcta catcgagggc agcacgctct acgacgctaa cggcgagcct        60
   tttgtcatgc gcggcatcaa ccacggccac gcctggtaca agcacgactc caacgtcgcc       120
   atccctgcta tcgccaacca gggcgctaac accatccgca tcgtcctcag cgacggtggc       180
   cagtgggcca aggacgacat caacacgctg aaccaggtcc tcgacctggc cgaggagcac       240
   gagatgatcg ctgtcgtcga ggtccacgac gctaccggca gcaactcgat ggccgacctc       300
   aaccgcgccg tcgactactg gatcgagatg aaggacgccc tgatcggcaa ggaagaccgc       360
   gtcatcatca acatcgccaa cgagtggtac ggcagctggg acggccaggg ctgggccaac       420
   ggctacaagg aagtcatccc ccgcctgcgc aacgctggct tcacccacac cctcatggtc       480
   gacgctgctg gctggggcca gtacccccag tcgatccacg actacggcca agaggtcttc       540
   aacgccgacc ctctggccaa caccatgttc agcatccaca tgtacgagta cgctggcggc       600
   aacgctgcta tggtccagtc gaacatcgac ggcgtcgtcg accagggcct cgctctggtc       660
   atcggcgagt tcggccacat gcacacggac ggcgacgtcg acgaggctac catcctgtcc       720
   tactcgcagc agcgcggcgt cggctggctc gcttggtcgt ggaagggcaa cggcacgcag       780
   tgggagtacc tcgacctgtc gtacgactgg cagggcacca acctcacctc gtggggcaac       840
   cgcatcgtcc acggccctaa cggcctcaaa gagacgtcga tccctagctc gatctttcac       900
   accgctccta acaacggcga ccctcctccc cacaacggca acgagacgat cctgtacgac       960
   ttcgagcacg gcacgcaggg ctggtcgggc tccagcctgc tgggcggccc ttggaccacc      1020
   aacgagtgga gcaccaacgg caaccactcg ctcaaggccg acatcttcct gagcgccaac      1080
   tcgaagcacg agctcgccaa ggtcgagaac cgcaacctct cgggctactc gacgctgcag      1140
   gctaccgtcc gccacgctca ctggggcaac gtcggcaacc tgacggctcg catgtacgtc      1200
   aagacgggct cgaactactc ctggttcaac ggcgacccca tccctgtcaa ctcggctaac      1260
   ggcaccaccg tcaccctccc tctgagctcc atccccaacc tcaacgacgt caaggagatc      1320
   ggcgtcgagt tcatcggcgc tagcaactcg aacggccaga ccgccatcta cctggaccac      1380
   gtcacgatcc agtag                                                        1395
<210> 24
   <211> 464
   <212> PRT
   <213> Artificial Sequence
<220>
   <223> variant
<400> 24
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
           35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ser Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ala Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Thr Gln Trp Glu Tyr Leu Asp Leu Ser Tyr Asp Trp Gln Gly
               260                 265                 270
   Thr Asn Leu Thr Ser Trp Gly Asn Arg Ile Val His Gly Pro Asn Gly
           275                 280                 285
   Leu Lys Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460
<210> 25
   <211> 1395
   <212> DNA
   <213> Artificial Sequence
<220>
   <223> variant
<210> 26
   <211> 464
   <212> PRT
   <213> Artificial Sequence
<220>
   <223> variant
<400> 26
   Gln Thr His Ser Gly Phe Tyr Ile Glu Gly Ser Thr Leu Tyr Asp Ala
   1               5                   10                  15
   Asn Gly Glu Pro Phe Val Met Arg Gly Ile Asn His Gly His Ala Trp
               20                  25                  30
   Tyr Lys His Asp Ser Asn Val Ala Ile Pro Ala Ile Ala Asn Gln Gly
               35                  40                  45
   Ala Asn Thr Ile Arg Ile Val Leu Ser Asp Gly Gly Gln Trp Ala Lys
       50                  55                  60
   Asp Asp Ile Asn Thr Leu Asn Gln Val Leu Asp Leu Ala Glu Glu His
   65                  70                  75                  80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ser Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ser Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Ala Gln Trp Glu Tyr Leu Asp Leu Ser Tyr Asp Trp Gln Gly
               260                 265                 270
   Thr Asn Leu Thr Ser Trp Gly Asn Arg Ile Val His Gly Pro Asn Gly
           275                 280                 285
   Leu Lys Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460